# EUROPEAN PATENT APPLICATION

(11) **EP 4 663 631 A1**
(43) Date of publication of application: **17.12.2025**
(21) Application number: 24752899.5
(22) Date of filing: 07.02.2024
(51) Int. Cl.: C07D 277/62, A61K 31/428, A61P 35/00, A61P 29/00, A61P 1/00, A61P 17/00, A61P 3/10, A61P 27/02, A61P 9/00

(54) **POLYMORPH AS PROTEIN KINASE MEK INHIBITOR, AND PREPARATION METHOD THEREFOR AND USE THEREOF**

(30) Priority: 10.02.2023 CN 202310099716; 22.01.2024 CN 202410090964
(71) Applicant: Shanghai Kechow Pharma, Inc., Shanghai 201203 (CN)
(72) Inventor: TIAN, Hongqi, Shanghai 201203 (CN); HUANG, Gongchao, Shanghai 201203 (CN)
(74) Representative: dompatent
(86) International application number: PCT/CN2024/076655
(87) International publication number: WO 2024/165044

(57) **Abstract**

The present invention relates to a polymorph of a benzothiazole compound as a protein kinase Mek inhibitor, and a preparation method therefor and the medical use thereof.

## Description

### FIELD OF THE INVENTION

The present invention relates to a polymorph of a benzothiazole compound as a protein kinase Mek inhibitor, and a preparation method therefor and the medical use thereof.

### BACKGROUND OF THE INVENTION

The overactivation of Ras/Raf/Mek/Erk signal transduction mechanism plays an important role in the proliferation and differentiation of cancer cells: it has been found that Ras/Raf/Mek/Erk signal transduction mechanism has been continuously activated or over-activated in many cancers, such as pancreatic cancer, colorectal cancer, lung cancer, bladder cancer, kidney cancer, skin cancer, breast cancer and so on. Inhibition of Ras/Raf/Mek/Erk signal transduction pathway is helpful to the treatment of these hyperproliferative diseases, in which Mek, which is located in the downstream target of Ras and Raf, plays a key role. The substrate of Mek phosphorylation is MAP kinase Erk. If Mek is suppressed, the Ras/Raf/Mek/Erk signal transduction pathway will be turned off and the proliferation of cancer cells will be inhibited. Therefore, Mek inhibitors can inhibit the growth of cancer cells, especially for cancers caused by excessive activation of Ras or Raf. Additionally, Mek also involves inflammatory diseases and symptoms, including acute and chronic inflammation.

Chinese patent application No.201210190520.4 discloses a variety of benzothiazole compounds, which show protein kinase Mek inhibitory activity, including 4-fluoro-5-(2-fluoro-4-iodoanilino)-N-(2-hydroxyethoxy)-1,3-benzothiazole-6-carboxamide (hereinafter referred to as compound 1). However, according to the preparation method of Chinese patent application No.201210190520.4, the solid form of compound 1 is amorphous. However, the purity of amorphous is usually difficult to control, physical and chemical properties of amorphous is usually unstable, and hygroscopicity of amorphous is poor. Moreover, the compound 1 is photodegraded and it is easy to degrade to form impurities (A), which affects the efficacy and storage stability of compound 1.

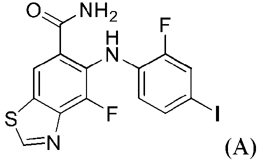

Therefore, it is necessary to develop a new polymorph form of the compound, which is beneficial to improve the purity control in the process production and improve the stability and storage stability of the compound.

### SUMMARY OF THE INVENTION

The invention provides polymorphism of compound 1, which has the advantages of high purity, good stability, good powder properties, mechanical stability and the like. More specifically, the invention provides seven crystalline forms of compound 1, namely, crystalline form I, crystalline form II, crystalline form IIIA, crystalline form IIIB, crystalline form IV, crystalline form V and VI, wherein the crystalline form I and IIIA show better solid state stability, mechanical stability, moisture absorption and light stability than other polymorph forms or amorphous forms, which is beneficial to the pharmaceutical process.

Specifically, the crystalline form I and IIIA of compound 1 exhibit one or more of the following advantages over amorphous forms:
1. The results of suspension competition show that the crystalline form I is more thermodynamically stable than the crystalline IIIA in the range from room temperature to 50 °C.
2. DVS results show that crystalline form I and crystalline form IIIA have almost no hygroscopicity, and there is no polymorph form transition after DVS test, while amorphous form changes to crystalline form I after DVS test.
3. The results of solid state stability showed that the homogeneous polymorph form did not change or the purity did not decrease after the crystalline form I was placed closed for 1 day at 60°C and open at 25°C/60%RH and 40°C/75%RH for 1 week, indicating that the crystalline form I has good physical and chemical stability under the evaluation conditions. The purity of crystalline form IIIA did not change after 1 day at 60°C, but the diffraction peak of crystalline form I was observed. After 1 week under 25° C/60%RH and 40°C/75%RH, the polymorph form did not change or the purity decreased. The purity of amorphous form did not change significantly under the three evaluation conditions, but the polymorph form changed to crystalline form I.
4. The results of light stability showed that the purity of crystalline form I was significantly higher than that of crystalline form IIIA, crystalline form V and amorphous under the condition of white light (5890 Lux + UV 8.7 W/m²) for 24 hours.
5. The results of dynamic solubility test of crystalline form I and amorphous in 1M HCl and pH 1.0/2.0/4.5/7.4 buffers at room temperature showed that the solubility of amorphous in all media was higher than that of crystalline form I within 10 minutes, and showed a process of rising and then decreasing, and the highest solubility measured in 1M HCl was higher than other pH buffers. Compared with amorphous, crystalline form I dissolves slowly in all media (different pH), and its solubility in 1m HCl is slightly higher than that in other pH buffers, thus ensuring that the dissolution of crystalline form I in vivo is more stable, and it is easier to obtain in vivo pharmacokinetics with stable blood concentration, which is helpful to avoid the risk of drug safety caused by excessive fluctuation of blood concentration.
6. The results of powder physical properties test showed that the crystalline form I and amorphous had similar fluidity, and the mechanical stability results showed that after tableting (350 MPa) and manual grinding (about 3 minutes), the crystalline form I did not undergo crystalline transformation and the crystallinity did not decrease significantly, the amorphous transformation to crystalline form I, and the crystalline form IIIA did not undergo polymorph form transformation, but the crystallinity decreased. and/or
7. In pharmacokinetic experiments, the higher exposure and plasma concentration of polymorph form I than polymorph form IIIA showed the potential to reduce the dose administered.

According to the characterization data and evaluation results, crystalline form I showed better light stability, and no polymorph form transition under all evaluation conditions, while amorphous was transformed into crystalline form I after DVS, solid stability, solubility and mechanical stability studies.

In one aspect, the present invention provides a polymorph of formula (I) 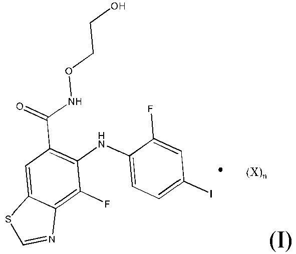 wherein n is 0 or 1, and X is acetonitrile, water,1,4-dioxane, ethanol, methanol, dimethylformamide, acetone or a mixture thereof.

In some embodiments, n is 0. In some embodiments, n is 1; X is acetonitrile, water, 1,4-dioxane, ethanol or dimethylformamide.

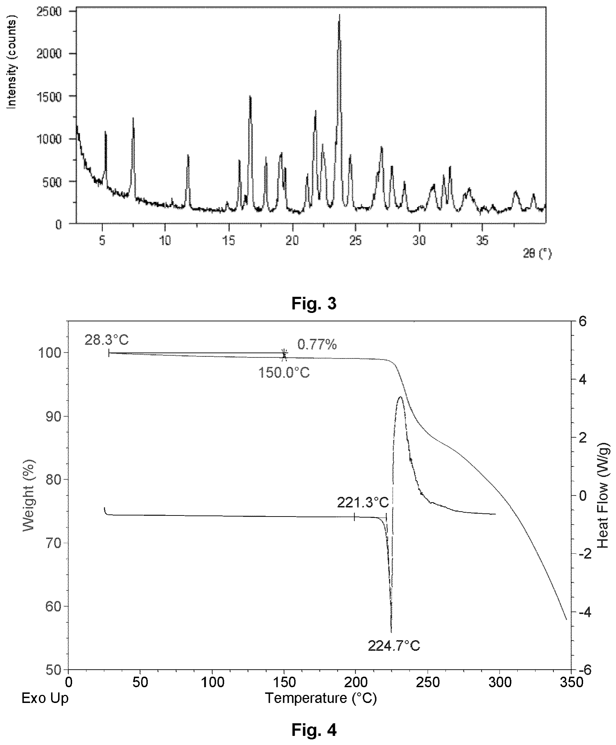

In some embodiments, n is 0 and the polycrystalline substance is crystalline form I, the characteristic XRPD diffraction peaks of the crystalline form I at the following 2θ positions: 16.71°±0.2°, 21.82°±0.2°and 23.75°±0.2°, using Cu-K α radiation. In some embodiments, the X-ray powder diffraction pattern of the crystalline form I also includes the characteristic diffraction peaks at the following 2θ positions: 7.48°±0.2°and 22.36°±0.2°. In some embodiments, the X-ray powder diffraction pattern of the crystalline form I also includes characteristic diffraction peaks at the following 2θ positions: 5.3°±0.2°, 24.57°±0.2°and 27.08°±0.2°. In some embodiments, the X-ray powder diffraction pattern of the crystalline form I also includes the characteristic diffraction peaks at the following 2θ positions: 11.81°±0.2°, 15.83°±0.2°, 17.92°±0.2°, 18.95°±0.2°and 19.17°±0.2°. In some embodiments, the X-ray powder diffraction pattern of the crystalline form I includes characteristic diffraction peaks at the following 2θ positions: 5.30°±0.2°, 7.48°±0.2°, 11.81°±0.2°, 14.85°±0.2°, 15.83°±0.2°, 16.71°±0.2°, 17.92°±0.2°, 18.95°±0.2°, 19.17°±0.2°, 19.43°±0.2°, 21.14°±0.2°, 21.82°±0.2°, 22.36°±0.2°, 23.75°±0.2°, 24.57°±0.2°, 27.08°±0.2°, 27.83°±0.2°, 28.88°±0.2°, 31.20°±0.2°, 31.92°±0.2°, 32.40°±0.2°, 33.91°±0.2°, 35.83°±0.2°, 37.51°±0.2°and 39.04°±0.2°. In some embodiments, the X-ray powder diffraction pattern of the crystalline form I is shown in Fig. 3. In some embodiments, the crystalline form I has TGA diagrams and/or DSC diagrams as shown in Fig. 4. In some embodiments, the crystalline form I is hydrate-free.

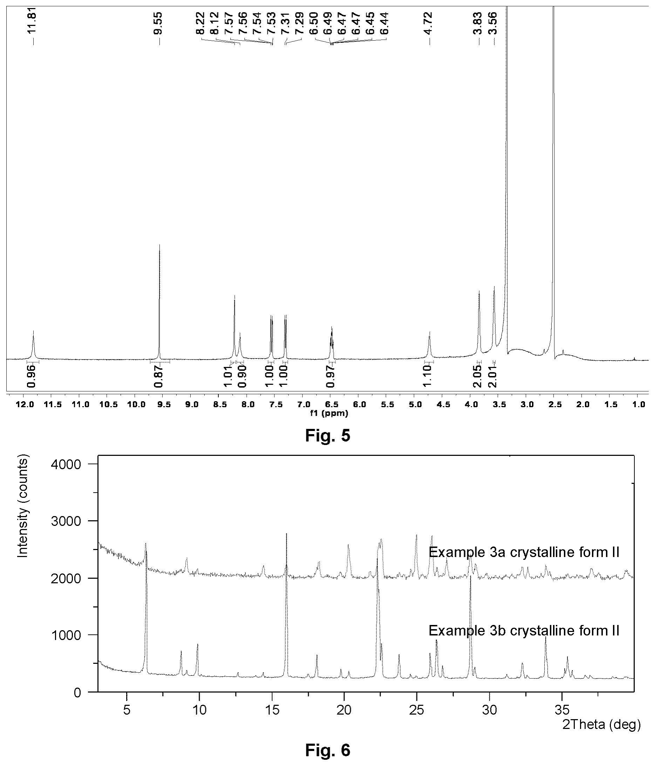

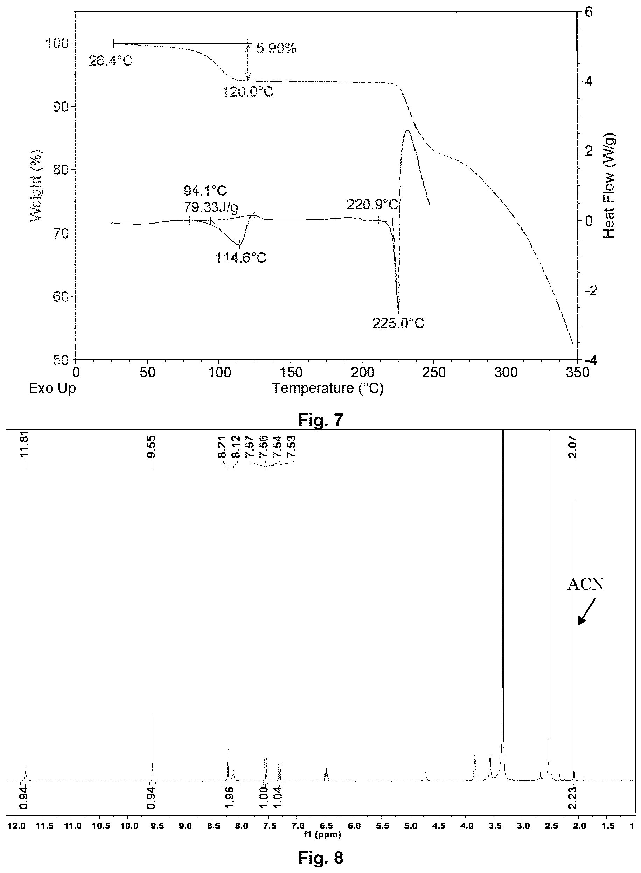

In some embodiments, n is 1, X is acetonitrile, and the polycrystalline substance is crystalline form II, the characteristic XRPD diffraction peaks of the crystalline form II at the following 2θ positions: 24.99°±0.2°, 26.05°±0.2°and 22.6°±0.2°, using Cu-K α radiation. In some embodiments, the X-ray powder diffraction patterns of the crystalline form II also include characteristic diffraction peaks at the following 2θ positions: 6.35°±0.2°, 20.34°±0.2°, 22.41°±0.2°and 28.71°±0.2°. In some embodiments, the X-ray powder diffraction patterns of the crystalline form II also include characteristic diffraction peaks at the following 2θ positions: 9.18°±0.2°, 16.03°±0.2°, 18.25°±0.2°, 27.07°±0.2°, 29.08°±0.2°and 33.93°±0.2°. In some embodiments, the X-ray powder diffraction patterns of the crystalline form II also include characteristic diffraction peaks at the following 2θ positions: 14.44°±0.2°, 24.64°±0.2°, 26.41°±0.2°, 32.27°±0.2°, 32.68°±0.2°, 37.07°±02°and 39.51°±0.2°. In some embodiments, the X-ray powder diffraction pattern of the crystalline form II includes characteristic diffraction peaks at the following 2 θ positions: 6.35°±0.2°, 9.18°±0.2°, 9.91°±0.2°, 14.44°±0.2°, 16.03°±0.2°, 18.25°±0.2°, 19.77°±0.2°, 20.34°±0.2°, 21.81°±0.2°, 22.41°±0.2°, 22.60°±0.2°, 23.84°±0.2°, 24.64°±0.2°, 24.99°±0.2°, 25.43°±0.2°, 26.05°±0.2°, 26.41°±0.2°, 26.41°±0.2°, 26.41°±0.2°. 27.07°±0.2°, 28.71°±0.2°, 29.08°±0.2°, 29.81°±0.2°, 31.16°±0.2°, 31.57°±0.2°, 32.27°±0.2°, 32.68°±0.2°, 33.93°±0.2°, 34.19°±0.2°, 35.42°±0.2°, 37.07°±0.2°, 37.56°±0.2°, 38.69°±0.2°and 39.51°±0.2°. In some embodiments, the X-ray powder diffraction pattern of the crystalline form II is shown in Fig. 6. In some embodiments, the crystalline form II has TGA diagrams and/or DSC diagrams as shown in Fig. 7.

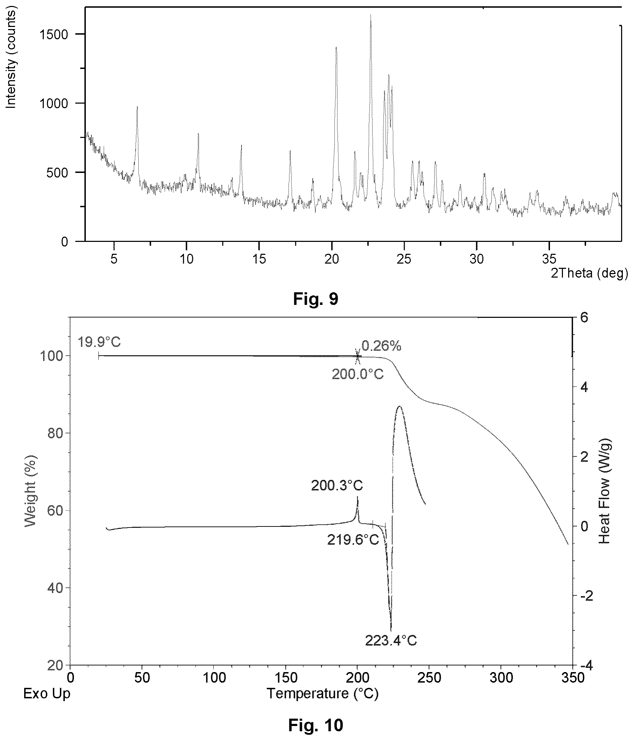

In some embodiments, n is 0, the polycrystalline substance is crystalline form IIIA, the characteristic XRPD diffraction peaks of the crystalline form IIIA at the following 2θ positions: 6.59°±0.2°, 22.69°±0.2°, 20.32°±0.2°, 23.62°±0.2°, 23.91°±0.2°and 24.15°±0.2°, using Cu-K α radiation. In some embodiments, the X-ray powder diffraction patterns of the crystalline form IIIA also include characteristic diffraction peaks at the following 2θ positions: 10.8°±0.2°, 17.14°±0.2°, 13.75°±0.2°, 21.59°±0.2°and 26.01°±0.2°. In some embodiments, the X-ray powder diffraction patterns of the crystalline form IIIA also include characteristic diffraction peaks at the following 2θ positions: 18.71°±0.2°, 21.97°±0.2°, 25.54°±0.2°, 27.13°±0.2°, 27.59°±0.2°and 30.51°±0.2°. In some embodiments, the X-ray powder diffraction pattern of the crystalline form IIIA includes characteristic diffraction peaks at the following 2θ positions: 6.59°±0.2°, 9.9°±0.2°, 10.8°±0.2°, 13.09°±0.2°, 13.75°±0.2°, 17.14°±0.2°, 17.87°±0.2°, 18.71°±0.2°, 19.19°±0.2°, 20.32°±0.2°, 21.59°±0.2°, 21.97°±0.2°, 22.69°±0.2°, 23.62°±0.2°, 23.91°±0.2°, 24.15°±0.2°, 25.54°±0.2°, 26.01°±0.2°, 27.13°±0.2°, 27.59°±0.2°, 28.83°±0.2°, 29.24°±0.2°, 30.51°±0.2°, 31.13°±0.2°, 31.79°±0.2°, 33.6°±0.2°, 34.14°±0.2°, 36.08°±0.2°, 36.67°±0.2° and 37.26°±0.2°. In some embodiments, the X-ray powder diffraction pattern of the crystalline form IIIA is shown in Fig. 9. In some embodiments, the crystalline form IIIA has TGA diagrams and/or DSC diagrams as shown in Fig. 10. In some embodiments, the crystalline form IIIA is hydrate-free.

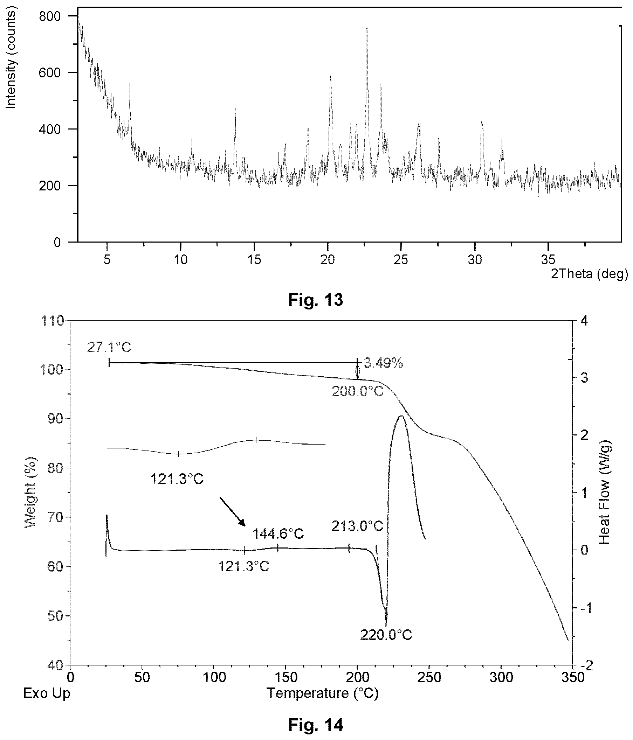

In some embodiments, n is 0, the polycrystalline substance is crystalline form IIIB, the characteristic XRPD diffraction peaks of the crystalline form IIIB at the following 2θ positions: 6.53°±0.2°, 13.69°±0.2°, 18.6°±0.2°, 20.19°±0.2°, 21.52°±0.2°and 22.64°±0.2°, using Cu-K α radiation. In some embodiments, the X-ray powder diffraction patterns of the crystalline form IIIB also include characteristic diffraction peaks at the following 2θ positions: 10.75°±0.2°, 17.07°±0.2°, 21.93°±0.2°, 26.13°±0.2°, 23.57°±0.2°and 30.46°±0.2°. In some embodiments, the X-ray powder diffraction patterns of the crystalline form IIIB also include characteristic diffraction peaks at the following 2θ positions: 13.05°±0.2°, 16.63°±0.2°, 20.82°±0.2°, 24.01°±0.2°, 27.55°±0.2°, 31.79°±0.2°. In some embodiments, the X-ray powder diffraction pattern of the crystalline form IIIB includes characteristic diffraction peaks at the following 2θ positions: 6.53°±0.2°, 10.75°±0.2°, 12.62°±0.2°, 13.05°±0.2°, 13.69°±0.2°, 16.63°±0.2°, 17.07°±0.2°, 18.60°±0.2°, 19.59°±0.2°, 20.19°±0.2°, 20.82°±0.2°, 21.52°±0.2°, 21.93°±0.2°, 22.64°±0.2°, 23.57°±0.2°, 24.01°±0.2°, 25.46°±0.2°, 26.13°±0.2°, 27.55°±0.2°, 30.46°±0.2°, 31.04°±0.2°, 31.79°±0.2°, 32.81°±0.2°, 33.54°±0.2°, 34.06°±0.2° and 34.46°+0.2°. In some embodiments, the X-ray powder diffraction pattern of the crystalline form IIIB is shown in Fig. 13. In some embodiments, the crystalline form IIIB has TGA diagrams and/or DSC diagrams as shown in Fig. 14.

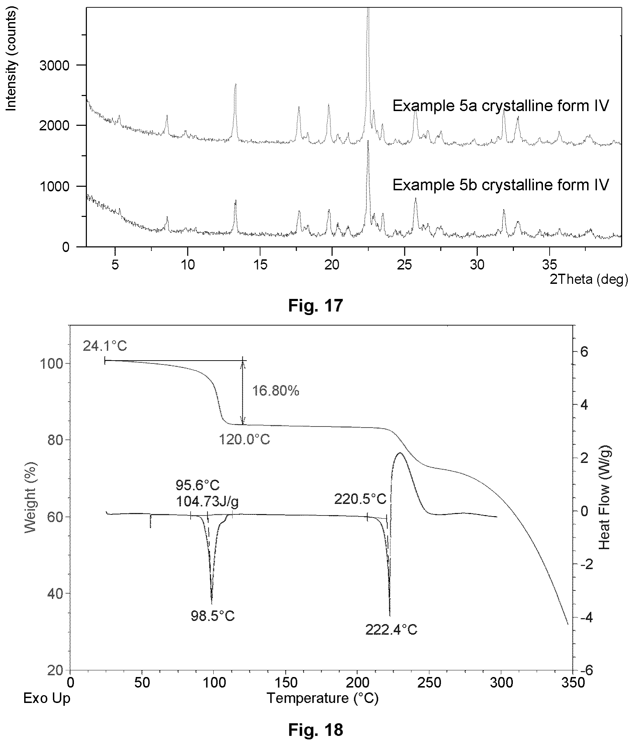

In some embodiments, n is 1, X is 14-dioxane, and the polycrystalline form is crystalline form IV, which is characterized in that the X-ray powder diffraction pattern of the crystalline form IV includes characteristic diffraction peaks at the following 2θ positions: 8.56°±0.2°, 13.29°±0.2°, 17.69°±0.2°, 19.75°±0.2°and 22.45°±0.2°, using Cu-K α radiation. In some embodiments, the characteristic XPRD diffraction peaks of the crystalline form IV at the following 2θ positions: 5.26°±0.2°, 18.29°±0.2°, 31.83°±0.2°, 25.68°±0.2°, 22.86°±0.2°, 32.81°±0.2°and 23.44°±0.2°. In some embodiments, the X-ray powder diffraction pattern of the crystalline form IV also includes characteristic diffraction peaks at the following 2θ positions: 126.57°±0.2°, 27.52°±0.2°, 35.69°±0.2°, 21.09°±0.2°, 20.35°±0.2°and 31.43°±0.2°. In some embodiments, the X-ray powder diffraction pattern of the crystalline form IV includes characteristic diffraction peaks at the following 2θ positions: 5.26°±0.2°, 8.56°±0.2°, 9.85°±0.2°, 13.29°±0.2°, 17.69°±0.2°, 18.29°±0.2°, 19.75°±0.2°, 20.35°±0.2°, 21.09°±0.2°, 22.45°±0.2°, 22.86°±0.2°, 23.44°±0.2°, 24.44°±0.2°, 25.68°±0.2°, 26.57°±0.2°, 27.52°±0.2°, 28.40°±0.2°, 29.78°±0.2°, 31.43°±0.2°, 31.83°±0.2°, 32.81°±0.2°, 34.29°±0.2°, 35.69°±0.2° and 37.72°±0.2°. In some embodiments, the X-ray powder diffraction pattern of the crystalline form IV is shown in Fig. 17. In some embodiments, the crystalline form IV has TGA diagrams and/or DSC diagrams as shown by Fig. 18.

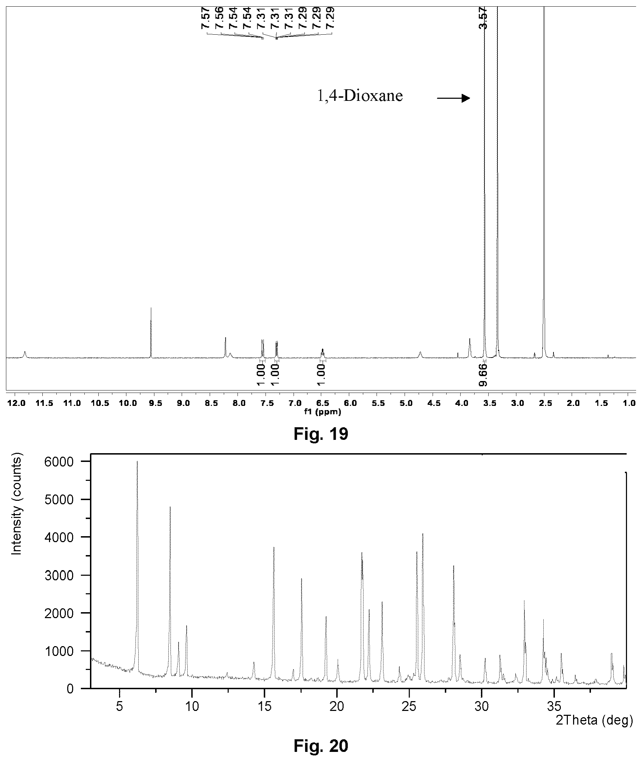

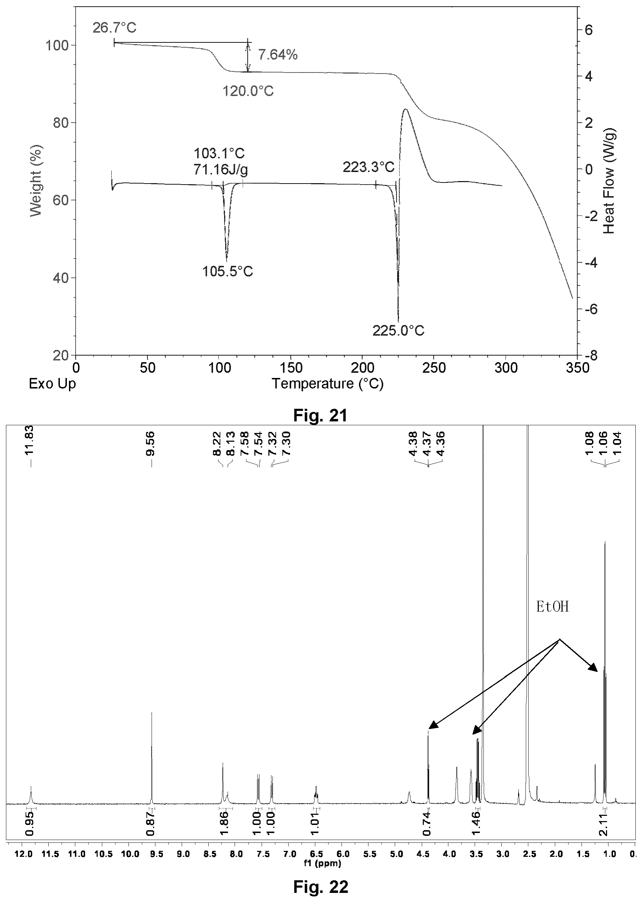

In some embodiments, n is 1, X is ethanol, and the polycrystalline form is crystalline form V, characteristic XPRD diffraction peaks of the crystalline form V at the following 2θ positions: 6.21°±0.2°, 8.47°±0.2°, 15.62°±0.2°, 21.73°±0.2°, 25.53°±0.2°, 25.94°±0.2°and 28.05°±0.2°, using Cu-K α radiation. In some embodiments, the X-ray powder diffraction pattern of the crystalline form V also includes the characteristic diffraction peaks at the following 2θ positions: 9.61°±0.2°, 17.55°±0.2°, 19.25°±0.2°, 22.22°±0.2°, 23.12°±0.2°, 32.92°±0.2°and 34.22°±0.2°. In some embodiments, the X-ray powder diffraction pattern of the crystalline form V also includes the characteristic diffraction peaks at the following 2θ positions: 9.06°±0.2°, 20.07°±0.2°, 28.49°±0.2°, 30.21°±0.2°, 31.25°±0.2°, 35.47°±0.2°and 38.94°±0.2°. In some embodiments, the X-ray powder diffraction pattern of the crystalline form V includes characteristic diffraction peaks at the following 2θ positions: 6.21°±0.2°, 8.47°±0.2°, 25.94°±0.2°, 15.62°±0.2°, 25.53°±0.2°, 28.05°±0.2°, 21.73°±0.2°, 17.55°±0.2°, 32.92°±0.2°, 23.12°±0.2°, 22.22°±0.2°, 19.25°±0.2°, 34.22°±0.2°, 9.61°±0.2°, 9.06°±0.2°, 38.94°±0.2°, 31.25°±0.2°, 35.47°±0.2°, 28.49°±0.2°, 30.21°±0.2°, 20.07°±0.2°, 39.78°±0.2°, 14.26°±0.2°, 24.32°±0.2°, 16.95°±0.2°, 32.33°±0.2°, 36.43°±0.2°, 24.94°±0.2°, 12.42°±0.2° and 37.86°±0.2°. In some embodiments, the X-ray powder diffraction pattern of the crystalline form V is shown in Fig. 20. In some embodiments, the crystalline form V has TGA diagrams and/or DSC diagrams as shown in Fig. 21.

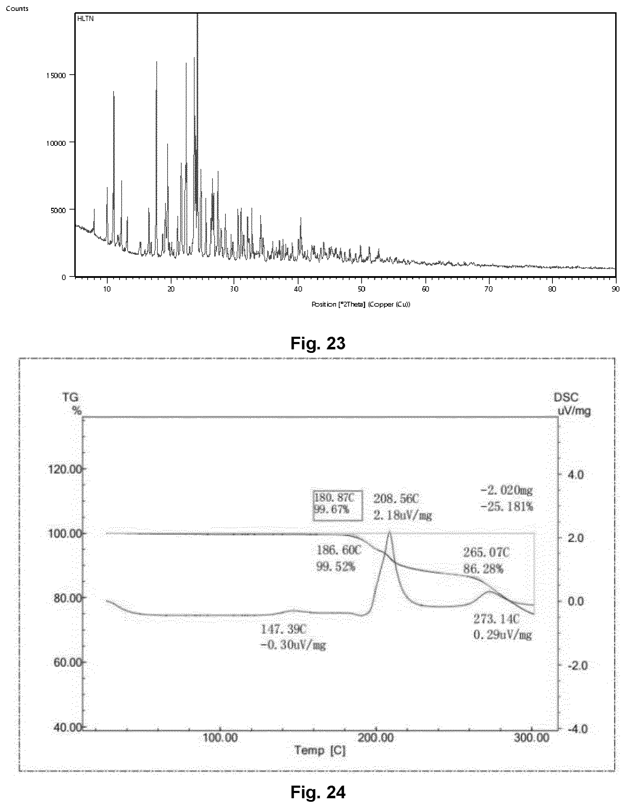

In some embodiments, n is 1, X is dimethylformamide, the polycrystalline form is crystalline form VI, and in some embodiments, the X-ray powder diffraction pattern of the crystalline form VI is shown in Fig. 23. In some embodiments, the TGA diagrams and / or DSC diagrams for crystalline form VI is shown in Fig. 24.

In some embodiments, crystalline form I, crystalline form II, crystalline form IIIA, crystalline form IIIB, crystalline form IV, crystalline form V and VI each have about 85% or more based on the weight of compound 1, for example, about 90% or more, for example, about 95% or more, for example, about 97% or more, for example, about 99% or more and include about 99.9% or higher purity, as determined by HPLC (high performance liquid chromatography). The rest of the material may contain the crystalline form of compound 1 and/or reactive impurities and/or processing impurities resulting from its preparation, such as photodegradable impurity compounds (A). The mixture of the crystalline form I of compound 1 and other solid forms (such as other polymorph forms and amorphous forms) is also within the scope of the present application.

In some embodiments, the polycrystalline form of compound 1 is essentially free of impurities (A). In some embodiments, the polycrystalline form of compound 1 contains less than 0.15 wt% impurity (A) relative to the polycrystalline form, e.g., 0.10%, 0.09%, 0.08%, 0.07%, 0.06%, 0.05%, 0.04%, 0.03%, 0.02%, or 0.01% wt% impurity (A).

In some embodiments, the crystalline form I of compound 1 is essentially free of impurities (A). In some embodiments, the crystalline form I of compound 1 contains impurities (A) less than 0.15 wt% relative to crystalline form I, e.g., 0.10%, 0.09%. 0.08%, 0.07%, 0.06%, 0.05%, 0.04%, 0.03%, 0.02% or 0.01% wt% impurity (A).

In some embodiments, crystalline form I, crystalline form II, crystalline form IIIA, crystalline form IIIB, crystalline form IV, crystalline form V and VI each have approximately 85% or more, e.g. approximately 90% or more, e.g. approximately 95% or more, e.g. approximately 97% or more, e.g. approximately 99% or more and include approximately 99.9% or more crystallinity.

In second aspect, the invention provides a pharmaceutical composition comprising any of the polycrystalline forms of the crystalline form I to the crystalline form VI of the invention, as well as a pharmaceutically acceptable carrier and/or excipient. In some embodiments, the pharmaceutical composition comprises a crystalline form I or IIIA of the present invention, as well as a pharmaceutically acceptable carrier and/or excipient. In some embodiments, the pharmaceutical composition comprises a crystalline form I of the present invention, as well as a pharmaceutically acceptable carrier and/or excipient.

In third aspect, the invention provides a method for treating mammalian tumors, chronic inflammatory diseases, inflammatory bowel diseases, skin diseases, diabetes mellitus, eye diseases, diseases related to mammalian angiogenesis or angiogenesis, diseases related to chronic pain and other diseases modulated by Mek cascade, and the method comprises administering any polymorph in the crystalline form I to crystalline form VI of the present invention to the mammalian; The present invention provides any polymorph in the crystalline form I to crystalline form VI of the present invention, which is used for the treatment of mammalian tumors, chronic inflammatory diseases, inflammatory bowel diseases, skin diseases, diabetes mellitus, eye diseases, diseases related to angiogenesis or angiogenesis in mammals, diseases associated with chronic pain and other diseases modulated by Mek cascades; Any polymorph in the crystalline form I to crystalline form VI of the present invention is used in the preparation of diseases for the treatment of mammalian tumors, chronic inflammatory diseases, inflammatory bowel diseases, skin diseases, diabetes mellitus, eye diseases, diseases related to angiogenesis or angiogenesis in mammals, diseases related to chronic pain and other diseases modulated by Mek cascade. In some embodiments, the mammals are humans.

In fourth aspect, the present invention provides a method for the treatment of RAS or RAF mutant cancer in mammals, including the administration of 4-fluoro-5-(2-fluoro-4-iodoanilino)-N-(2-hydroxyethoxy)-1,3-benzothiazole-6-carboxamide (compound 1) or its medicinal salt to the mammal. In some embodiments, the compound 1 is any of the polymorphs from crystalline form I to crystalline form VI. In some embodiments, the RAS or RAF mutant cancer is, for example, KRAS mutant cancer, NRAS mutant cancer, HRAS mutant cancer or BRAF mutant cancer. In some embodiments, the RAS mutant cancer is pancreatic cancer, colorectal cancer, lung cancer, melanoma, acute myeloid leukemia, bladder cancer or head and neck cancer, etc. In a preferred embodiment, the cancer is a NRAS mutant cancer. In some embodiments, the NRAS mutant cancer is a melanoma with RNAS mutation.

In one embodiment, KRAS includes mutations at one or more locations selected from codons 12, 13, 59, and 61. In one embodiment, the KRAS mutant form has mutations at one or more amino acid locations selected from G12, G13, S17, P34, A59, and Q61. In an embodiment, the KRAS mutant form has one or more amino acid substitutions selected from: G12C, G12S, G12R, G12F, G12L, G12N, G12A, G12D, G12V, G13C, G13S, G13D, G13V, G13P, S17G, P34S,A59E,A59G,A59T, Q61K, Q61L, Q61R, and Q61H. In one embodiment, the KRAS mutant form has mutations at one or more amino acid positions selected from G12, G13, A59, Q61, K117 and A146. In an embodiment, the KRAS mutant form has one or more amino acid substitutions selected from: G12C, G12R, G12S, G12A, G12D, G12V, G13C, G13R, G13S, G13A, G13D, G13V, A59E,A59G, A59T, Q61K, Q61L, Q61R, Q61H, K117N, K117R, K117E, A146P, A146T and A146V. In one embodiment, the BRAF mutation is a BRAF V600E mutation.

In one embodiment, NRAS includes mutations at one or more locations selected from codons 12, 13, 59, 61, and 146. In some embodiments, the NRAS mutant form has mutations at one or more amino acid positions selected from G12, G13, A59, Q61, K117, and A146. In some embodiments, the NRAS mutant form has one or more amino acid substitutions selected from: G12C, G12R, G12S, G12A, G12D, G12V, G13C, G13R, G13S, G13A, G13D, G13V, A59D, A59T, Q61K, Q61L, Q61R, Q61H, K117N, K117R, K117E, A146P, A146T, and A146V.

In some embodiments, the cancer in question is an early, intermediate, or advanced stage cancer. Cancer can be locally advanced or metastatic. In some embodiments, the mammal has previously received immunotherapy. In some embodiments, the mammal has previously received immunotherapy and has NRAS-mutated advanced melanoma. In some embodiments, the melanoma is selected from: advanced melanoma, unresectable melanoma, metastatic melanoma, melanoma with BRAF mutation, melanoma with NRAS mutation, cutaneous melanoma, or intraocular melanoma.

In some embodiments, Compound 1 is in the form of tablets, powders, granules, patches, inhalers, and capsules. In some embodiments, Compound 1 is in the form of a capsule. In some embodiments, Compound 1 is administered at a dose of 5-50 mg once or twice daily. In some embodiments, Compound 1 is administered at a dose of 12 mg twice daily.

In a fifth aspect, the present invention provides a method for preparing the crystalline Form I of the present invention, which method includes any of the following:
a) Adding an amorphous sample of Compound 1 to the solvent and then heating it at a temperature above about 70 °C to obtain a clear solution. Cool the liquid to room temperature, keep it at room temperature and continue stirring until the solid precipitates, filter and dry; or
b) Dissolve the amorphous sample of compound 1 in a good solvent, filter to obtain a clear solution, and add an anti-solvent while stirring the clear solution until solid precipitates; or
c) Dissolve the amorphous sample of Compound 1 in the solvent, stir at about 50 °C, then filter the filtrate, cool the filtrate to about 5 °C, and collect the precipitated solid.

In some embodiments, the heating temperature in Method 1) is about 75 °C to about 100 °C; about 80 °C to about 90 °C; or about 75 °C or about 85 °C. In some embodiments, the room temperature in Method 1) is about 20 - about 25 °C. In some embodiments, the time to continue stirring in Method 1) is about 0.5 -12 hours, about 1-12 hours, about 1- 8 hours, about 1- 5 hours, or longer, or the time to continue stirring is about 24 -76 hours. In some embodiments, the clear liquid in Method 1) is cooled to room temperature within about 2 to about 5 hours or about 2.5-about 3 hours. In some embodiments, the room temperature in the method 1) continues to stirring, and the temperature can be further reduced to about 0- about 10 °C, and the heat preservation is stirred. In some embodiments, the heat preservation of about 0-about 10 °C is about 1-7 hours, about 1-about 8 hours, about 1-about 5 hours or about 0- about 10 °C. In some embodiments, the solvents in Method 1) are water, methanol, ethanol, isopropanol, acetone, methyl isobutyl ketone, 2-butanone, ethyl acetate, isopropyl acetate, methyl tert-butyl ether, tetrahydrofuran, anisole ether, 2-methyltetrahydrofuran, cyclopentylmethyl ether, 1,4-dioxane, acetonitrile, dichloromethane, toluene, m-xylene, n-heptane, n-hexane, n-pentane, dimethyl sulfoxide, dimethylacetamide, N-methylpyrrolidone or a mixture thereof. In some embodiments, the solvent described in Method 1) is ethanol.

In some embodiments, the good solvent in Method 2) is a solvent in which Compound 1 is soluble, and the antisolvent in Method 2) is a solvent in which Compound 1 is insoluble. In some embodiments, the good solvent in Method 2) is MEK, 1,4-dioxane, or DMSO. In some embodiments, the antisolvents in Method 2) is MTBE, EtOAc, CHCl₃, n-heptane, Anisole, EtOAc, H₂O, IPAc, CPME, DCM, or toluene.

In some embodiments, the cooling rate described in Method 3) is about 0.1 °C/min. In some embodiments, the solvent of method 3) is MIBK, Methyl acetate, 2-MeTHF or acetone / EtOH (1: 1).

The present invention provides a method for preparing the crystal form V of the invention. The method includes: adding an amorphous sample of Compound 1 to a solvent, and then heating at a temperature below about 70 °C, and the resulting clear liquid is cooled to about 0 to about 10 °C. Let stand at about 5 °C until the solid precipitates and dry. In some embodiments, the method heats at about 70 °C, about 60 °C, or about 50 °C. In some embodiments, the cooling rate is about 0.1 to about 0.5°C/min. In some embodiments, the cooling rate is about 0.1°C/min. In some embodiments, the resulting clear liquid is cooled to about 5 °C.

The present invention provides a method for preparing the crystalline Form IIIA of the invention. The method includes: heating the crystalline Form II to a first temperature and holding it at a constant temperature for about 3 to about 10 minutes, and then cooling to obtain the crystalline Form IIIA. In some embodiments, the first temperature is from about 100 to about 140 °C; such as from about 110 to about 130 °C, such as about 120 °C. In some embodiments, the Form II is thermostated at the first temperature for about 5 minutes. In some embodiments, the Form II is isothermalized and then cooled to room temperature to about 50 °C, for example, to room temperature.

Differential scanning calorimetry (DSC) is well known in this field. The melting peak height of the DSC curve depends on many factors related to sample preparation and instrument geometry, while the peak position is relatively insensitive to experimental details. Therefore, in some embodiments, the crystalline compound of the present invention has a DSC diagram with a characteristic peak position, has substantlly the same properties as the DSC diagram provided in the drawings of the invention, and the error tolerance of the measured value is within ±5 °C, which is generally required within ±3 °C.

The numerical values described and claimed herein are approximations. Variations within Values may be due to equipment calibration, equipment error, crystal purity, crystal size, sample size, and other factors.

The crystal form of the present invention is not limited to the characteristic maps exactly the same as those described in the drawings disclosed by the invention, such as XRPD, DSC, TGA, DVS, isothermal adsorption curves, and any has substantially the same characteristics as those depicted in the drawings. Any crystalline form with the same or essentially the same characteristic pattern falls within the scope of the invention.

### TERMS

Unless otherwise specified in this document, all other technical and scientific terms used in this application have meanings generally understood by those skilled in the field to which the invention belongs.

This application includes singular words such as "one", "one" and "stated" used in the claim, including their corresponding plural references, unless the contrary is clearly indicated in the context. Thus, for example, the reference to "polymorph form" includes one or more such different polymorph forms and the reference to "said method" includes a reference to equivalent steps and methods known to those of ordinary skill in the art, which may be improved or replaced by the methods described in this application.

In the following entire specification and claims, unless otherwise required by the context, the terms "contains" and variations such as "contains" and "includes" will be construed to implicitly include said integer or step or set of integers or steps, but not to the exclusion of any other integer or step or set of integers or steps. When used in this application, the term "contains" may be replaced by the term "contains" or, in some cases, with the term "has".

The term "about" refers to 10% or 5% or 2% of the value added or subtracted from the specified item.

"Therapeutic effective dose" means the amount of compounds that cause the physiological or medical response of the tissue, system, or subject, which is sought, includes the amount of compounds administered to the person being treated that are sufficient to prevent the occurrence of one or more symptoms of the disease or disease under treatment or to alleviate it to a certain extent. The "therapeutic effective dose" may vary with the symptoms of the compound, disease, disease, and / or disease or disease, the severity of the disease or disease, and / or the symptoms of the disease or disease, the age of the subject to be treated, and / or the weight of the subject to be treated. The appropriate equivalent in any given case is obvious to those skilled in the art or can be determined by routine experiments. In the case of combination therapy, the "therapeutic effective dose" refers to the total number of combined objects that effectively treat a disease, disease, or condition.

"Excipient" means an excipient that is not a therapeutic agent but is used as a diluent, excipient, binder and/or vehicle for addition to a pharmaceutical composition to improve its disposal or storage properties or to permit or facilitate the formation of a compound or pharmaceutical composition into a unit dosage form for administration.

"Polymorph form" or "crystal" or "polycrystalline form" refers to any solid substance in three-dimensional order, which produces a characteristic XRPD pattern with well-defined peaks, as opposed to amorphous solid matter.

"Amorphous" refers to amorphous molecular and/or ionic solid forms. Amorphous solids do not show X-ray diffraction patterns with sharp maxima.

"Hydrate" refers to the crystalline form of a molecule, which further contains water incorporated into the crystalline structure. The water molecules in the hydrate can exist in a regular arrangement and/or disordered arrangement. Hydrates can contain either stoichiometric or non-stoichiometric water molecules.

"Anhydrate" refers to a crystal that contains essentially no water molecules of any kind, such as a crystalline form in which the crystal lattice or unit cell contains no water molecules at all.

The term "solvent complex" refers to the crystalline form of a molecule which further contains one or more solvent molecules incorporated into the crystalline structure. Solvent molecules in solvates can exist in regular and/or disordered arrangements. Solvates can contain either stoichiometric or non-stoichiometric solvent molecules. Exemplary solvates include, but are not limited to, hydrates, ethanol salts, methanol salts, and isopropanol salts, acetic acid. Methods of solvation are generally known in the art. It is important to note that in solvated substances, the substance bound to the main molecule (for example, the active pharmaceutical ingredient) is liquid at room temperature, whereas in eutectic, the substance is solid at room temperature.

The polymorph form disclosed in this application is basically pure crystal. The term "substantially pure" as used in this application means a polymorph form disclosed in the present application that is at least 85 wt%, preferably at least 95 wt%, and preferably at least 99 wt%, and also includes a polymorph equal to approximately 100 wt%. The remaining materials include one or more other forms of the compound and/or reactive and/or processing impurities resulting from their preparation. For example, the crystalline form of compound 1 may be considered essentially pure because it has a purity greater than 90 wt%, as measured by means known and generally accepted in the art at the time, where the remaining material less than 10 wt% contains amorphous and/or one or more other forms and/or reactive and/or processing impurities of compound 1.

"X-ray powder diffraction pattern (XRPD pattern)" refers to the diffraction pattern of experimental observations or the parameters, data or values derived from them. XRPD spectra are typically characterized by peak position (abscissa) and/or peak intensity (ordinate). For the polymorph forms disclosed in this application, only the main peaks (i.e., the most characteristic, significant, distinctive, and/or reproducible peaks) are summarized; other peaks can be obtained from diffraction patterns using conventional methods. The above main peaks can be repeated within the error bounds (±2 in the decimal places given at the end, or 0.2 ± the value given).

"20" refers to the peak position in degrees (°) set based on X-ray diffraction experiments and is usually the abscissa unit in the diffraction spectrum. If the reflection is diffracted when the incident beam forms an angulation angle with a lattice plane, the experimental setup requires recording the reflected beam at an angle of 2θ. The specific 2θ value of the polymorph form mentioned in the present application is intended to indicate the 2θ value (expressed in degrees) measured using the X-ray diffraction experimental conditions described in the present application.

For X-ray diffraction peaks, the term "essentially the same" or "essentially as shown in Figure XX" refers to the consideration of representative peak positions and intensity variations. For example, those skilled in the art will understand that the peak position (2θ) will show some variation, usually as much as 0.1-0.2°, and that the instrument used to measure diffraction will also cause some variation. In addition, those skilled in the art will understand that the relative peak intensity will vary depending on the differences between instruments, as well as the degree of crystallinity, preferred orientation, the surface of the prepared sample, and other factors known to those skilled in the art and should be regarded as qualitative measurements only.

Pharmaceutical compositions containing the compounds disclosed in this application may be administered orally, inhaled, rectal, parenteral, or topically to subjects who need it. For oral administration, the drug composition can be a regular solid preparation such as tablets, powders, granules, capsules, etc., a liquid preparation such as water or oil suspension, or other liquid preparations such as syrup, solution, suspension, etc.; For parenteral administration, the drug composition can be solution, aqueous solution, oil suspension concentrate, lyophilized powder, etc. Preferably, the formulation of the composition of the drug is selected from tablets, coated tablets, capsules, suppositories, nasal sprays, or injections, preferably tablets or capsules. The drug composition can be given as a single unit with a precise dose. In addition, the composition of the drug can further contain additional active ingredients.

All preparations of the pharmaceutical compositions disclosed in this application can be prepared by conventional methods in the pharmaceutical field. For example, the active ingredient may be mixed with one or more excipients to prepare the desired preparation. "Pharmaceutical acceptable excipient" means conventional drug carriers suitable for the desired pharmaceutical preparations, such as diluents, media such as water, various organic solvents, fillers such as starch, sucrose, adhesives such as cellulose derivatives, alginate, gelatin and polyvinylpyrrolidone (PVP); wetting agents such as glycerol; disintegrants such as Agar, calcium carbonate and sodium bicarbonate; absorption enhancers such as quaternary ammonium compounds. Surfactants such as hexadecyl alcohol; absorption carriers such as kaolin and soap clay; lubricants such as talc powder, calcium stearate, magnesium stearate, polyethylene glycol, etc. In addition, the pharmaceutical composition further includes other pharmaceutically acceptable excipients such as dispersants (decentralized agent), stabilizers, thickeners, complexing agents, buffers, penetration enhancers, polymers, aromatic compounds, sweeteners, and dyes.

"Pharmaceutical composition" means a composition consisting of a combination of a polymorph form of a compound of the present invention and at least one other pharmaceutically acceptable carrier. "Pharmaceutically acceptable carriers" means media commonly accepted in the art for the delivery of bioactive agents to animals, in particular mammals, including, i.e., adjuvants, excipients or vehicles such as diluents, preservatives, fillers, flow regulators, disintegrants, wetting agents, emulsifiers, suspensions, sweeteners, flavorings, fragrances, antibacterial agents, antifungal agents, lubricants and dispensing agents, depending on the mode of application and the nature of the dosage form.

Pharmaceutically acceptable carriers are formulated according to many factors within the cognitive range of ordinary technicians in the field. These include, but are not limited to: the type and nature of the active agent being prepared; the subject to be given of the composition containing the drug; the expected route of application of the composition; and the therapeutic indications being targeted. Pharmaceutically acceptable carriers include both aqueous and non-aqueous liquid media, as well as a variety of solid and semi-solid dosage forms. Such carriers may also include many different ingredients and additives other than surfactants, which are included in the formulation for a variety of reasons familiar to ordinary technicians in the art (for example, stabilization of active agents, adhesives, etc.). Descriptions of suitable pharmaceutically acceptable carriers and the factors involved in their selection are available from a variety of readily available sources such as Allen, Jr., L.V. et al., Remington: The Science and Practice of Pharmacy (volume 2), 22nd edition, Pharmaceutical Press (2012).

Of course, the dosing regimen in solid form of this application will vary according to known factors such as: the pharmacodynamic profile of a particular agent and the manner and route of its administration; recipient's species, age, sex, health, medical condition, and weight; the nature and extent of symptoms; types of concurrent treatments; frequency of treatment; Route of administration, renal and hepatic function of the patient and desired effect. Typically, the daily oral dose range for each active ingredient will be between about 0.001 and about 5000 mg/day, preferably between about 0.01 and about 1000 mg/day, and preferably between 0.1 and about 250 mg/day. Intravenously, during a constant rate infusion, the optimal dose will range from approximately 0.01 to approximately 10 mg/kg/minute. The compound of the present invention may be administered in a single daily dose, or the total daily dose may be administered in divided doses of two, three or four times per day.

The dosage form (pharmaceutical composition) for application may contain active ingredients ranging from about 1 mg to about 2000 mg per dose unit. In these pharmaceutical compositions, the active ingredient will normally be present in an amount of about 0.1% by weight based on the total weight of the composition.

### BRIEF DESCRIPTION OF DRAWINGS

Fig. 1 shows an XRPD diagram for the amorphous polymorph of compound 1.
Fig. 2 shows dynamic water adsorption (DVS) profile for the amorphous polymorph of compound 1.
Fig. 3 shows XRPD diagram of polymorph form I of example 2a.
Fig. 4 shows TGA/DSC thermogram of crystalline form I in embodiment 2a.
Fig. 5 shows ¹H NMR diagram of crystalline form I in example 2a.
Fig. 6 shows the XRPD diagram of the crystalline form II of example 3a and 3b.
Fig. 7 shows the TGA/DSC thermogram of the crystalline form II of example 3a.
Fig. 8 shows an ¹H NMR diagram of the crystalline form II of example 3a.
Fig. 9 shows an XRPD diagram of the crystalline form IIIA of example 4a.
Fig. 10 shows an embodiment TGA/DSC thermogram of 4a crystal IIIA.
Fig. 11 shows an ¹H NMR diagram of 4a crystal IIIA.
Fig. 12 shows an example of XRPD overlay of 4a crystal IIIA before and after heating.
Fig. 13 shows an embodiment XRPD diagram of a 4b crystal IIIB.
Fig. 14 shows an TGA/DSC thermogram of a 4b crystal IIIB.
Fig. 15 shows an embodiment ¹H NMR diagram of 4b crystal IIIB
Fig. 16 shows the XRPD diagram of crystalline form IIIB under changed temperature in example 4b;
Fig. 17 shows the XRPD diagram of crystalline form IV in example 5a and 5b.
Fig. 18 shows the TGA/DSC thermogram of crystalline form IV in example 5a.
Fig. 19 shows the ¹HNMR diagram of crystalline form IV in example 5a.
Fig. 20 shows the XRPD diagram of crystalline form V in Example 6a.
Fig. 21 shows the TGA/DSC thermogram of crystalline form V in Example 6a.
Fig. 22 shows the ¹H NMR spectrum of crystalline form V in Example 6a.
Fig. 23 shows the XRPD diagram of crystalline form VI in Example 7a.
Fig. 24 shows the DSC/TGA thermogram of crystalline form VI in Example 7a.
Fig. 25 shows the XRPD diagram of crystalline form IV before and after heating in Example 5a.
Fig. 26 shows the XRPD stack diagram of crystalline form II in Example 3a before and after heating.
Fig. 27 shows the XRPD diagram of crystalline form II in Example 3b before and after heating.
Fig. 28 shows the XRPD diagram of crystalline form V before and after heating in Example 6a.
Fig. 29 shows the XRPD overlay of crystalline forms I and IIIA competing in EtOAc suspension.
Fig. 30 shows the XRPD overlay of crystalline forms I and IIIA competing in MIBK suspension.
Fig. 31 shows the XRPD stacking diagram (I/II) of competitive suspension of crystalline forms I and IIIA in acetone/_{H2O}.
Fig. 32 shows the XRPD stacking diagram (II/II) of competitive suspension of crystalline forms I and IIIA in acetone/_{H2O}.
Fig. 33 shows the DVS isotherm of crystalline form I.
Fig. 34 shows the XRPD overlay of crystalline form I before and after DVS testing.
Fig. 35 shows the XRPD overlay of amorphous samples before and after DVS testing.
Fig. 36 shows the DVS isotherm of crystalline form IIIA in the humidity experiment.
Fig. 37 shows the XRPD overlay of crystalline form IIIA before and after DVS testing.
Fig. 38 shows the XRPD overlay before and after the solid-state stability evaluation of crystalline form I in Example 2a.
Fig. 39 shows the XRPD overlay before and after the evaluation of the stability of amorphous solids.
Fig. 40 shows the XRPD overlay before and after the solid-state stability evaluation of crystalline form IIIA.
Fig. 41 shows the solubility of amorphous materials at different pH values (with a feed concentration of 0.25 mg/mL).
Fig. 42 shows the solubility diagram of crystalline form I at different pH values (with a feed concentration of 0.25 mg/mL).
Fig. 43 shows the solubility diagrams of amorphous and crystalline forms I at different pH values (with a feed concentration of 0.05 mg/mL).
Fig. 44 shows the XRPD overlay of crystalline form I before and after grinding.
Fig. 45 shows the XRPD overlay of crystalline form I before and after lamination.
Fig. 46 shows the XRPD overlay of crystalline form IIIA before and after grinding.
Fig. 47 shows the XRPD stacking diagram of crystalline form IIIA before and after lamination.
Fig. 48 shows the XRPD overlay before and after amorphous grinding.
Fig. 49 shows the XRPD overlay before and after amorphous pressing.
Fig. 50 shows the XRPD stack diagram for evaluating the light stability of crystalline form I.
Fig. 51 shows the XRPD overlay for evaluating the light stability of crystalline form IIIA.
Fig. 52 shows the XRPD stack diagram for evaluating the light stability of amorphous samples.
Fig. 53 shows the XRPD stack diagram for evaluating the light stability of crystalline form V.
Fig. 54 shows the HPLC plot for evaluating the light stability of crystalline form I.
Fig. 55 is an HPLC plot for evaluating the light stability of crystalline form IIIA.
Fig. 56 is an HPLC plot for evaluating the light stability of amorphous samples.
Fig. 57 is an HPLC plot for evaluating the stability of crystalline form V under light irradiation.

### Examples

The following embodiments are only used to exemplify the invention and do not limit the invention in any way.

### Solvent name corresponding table

| Abbreviations | Full names | Abbreviations | Full names |
|---|---|---|---|
| MeOH | Methanol | CPME | Cyclopentyl methyl ether |
| EtOH | Ethanol | | |
| IPA | Isopropanol | ACN | Acetonitrile |
| | | DCM | Dichloromethane |
| MIBK | 4-Methyl-2-pentanone | | |
| MEK | 2-Butanone | | |
| EtOAc | Ethyl acetate | | |
| IPAc | Isopropyl acetate | | |
| MTBE | tert-Butyl methyl ether | | |
| THF | Tetrahydrofuran | DMSO | Dimethyl sulfoxide |
| | | DMAc | N,N-dimethylacetamide |
| 2-MeTHF | 2-Methyltetrahydrofuran | NMP | N-methyl-2-pyrrolidone |
| DMF | N,N-dimethylformamide | | |

The following instruments and methods are adopted in the present invention: Instruments and methods

X-ray powder diffraction (XRPD): The XRPD results are collected on X'Pert³ and Empyrean X-ray powder diffraction analyzers, and the scanning parameters are shown in Table 1.

**Table 1: XRPD test parameters**

| Pattern | Conventional reflection |
|---|---|
| X-ray | Cu, kα; Kα1 (Å): 1.540598, Kα2 (Å): 1.544426 |
| | Strength ratio Kα2/Kα1: 0.50 |
| X-ray tube setting | 45 kV, 40 mA |
| Divergent slit | 1/8° |
| Scanning mode | Continuous |
| Scanning range (°2TH)3° | ~ 40° |
| Scanning step (°2TH) | 0.0263 |
| Scan time per step (s) | 46.67 |
| Scanning time (s) | ~5 min |

Thermogravimetric analysis (TGA) and differential scanning calorimetry (DSC): TGA and DSC were collected on TA Discovery 5500 thermogravimetric analyzer and TA Discovery 2500 differential scanning calorimeter, respectively. Table 2 lists the test parameters of TGA and DSC.

**Table 2:TGA and DSC test parameters**

| Parameters. | TGA | DSC |
|---|---|---|
| Method | Linear temperature increase | Linear temperature increase |
| Sample plate | Aluminum plate, open | Aluminum plate, gland |
| Temperature range | Room temperature ~ 350 °C | 25 °C ~ 300 °C |
| Heating rate | 10 °C/min | 10 °C/min |
| Protective gas | Nitrogen | Nitrogen |

Liquid nuclear magnetic resonance (1H Solution NMR): hydrogen liquid nuclear magnetic resonance was collected on Bruker 400M nuclear magnetic resonance with DMSO-d6 as solvent.

### Dynamic water adsorption (DVS):

The dynamic water adsorption (DVS) curve was collected on the DVS Intrinsic of SMS (Surface Measurement Systems). The relative humidity at 25 °C is corrected by the tidal point of LiCl, Mg(NO₃)₂ and KCl. The DVS test parameters are listed in Table 3.

**Table 3:DVS test parameters**

| Parameters. | DVS |
|---|---|
| Temperature | 25 °C |
| Sample quantity | 10-30 mg |
| Protective gas and flow rate | N2, 200 mL/min |
| dm/dt | 0.002%/min |
| Minimum dm/dt equilibrium time | 10 min |
| Maximum equilibrium time | 180 min |
| RH Range | 0%RH~95%RH~0%RH (Crystalline formI) |
| | Room humidity ~95%RH~0%RH~95%RH (Amorphous) |
| RH Gradient | 10%RH (0%RH~90%RH & 90%RH~0%RH) |
| | 5%RH (90%RH~95%RH & 95%RH~90%RH) |

High performance liquid chromatography (UPLC): the purity and solubility in the experiment were tested by Agilent 1290 ultra high performance liquid chromatography. The analytical conditions are shown in tables 4 and 5.Table 4: ultra-high performance liquid chromatography test conditions for purity testing

| UPLC | Agilent 1290 (DAD detector) | |
|---|---|---|
| Chromatographic column | Xbridge Shield RP18, 150×4.6 mm, 5 µm | |
| Mobile phase | A: 10 mM NH4Ac (pH=8.5):ACN = 95:5 (v/v) | |
| | B: 10 mM NH4Ac (pH=8.5):ACN = 2:8 (v/v) | |
| Elution gradient | Time (min) | %B |
| | 0.0 | 20 |
| | 23.0 | 80 |
| | 35.0 | 80 |
| | 35.1 | 20 |
| | 45.0 | 20 |
| Running time | 45.0 min | |
| Post-run time | 0.0 min | |
| Mobile phase flow rate | 1.0 mL/min | |
| Injection volume | 5 µL | |
| Detection wavelength | 270 nm | |
| Column temperature | 40 °C | |
| Injector temperature | Room temperature | |

**Table 5: ultra-high performance liquid chromatography test conditions for solubility test**

| UPLC | Agilent 1290 (DAD detector) | |
|---|---|---|
| Chromatographic column | Xbridge Shield RP18, 150×4.6 mm, 5 µm | |
| Mobile phase | A: 10 mM NH4Ac (pH=8.5):ACN = 95:5 (v/v) | |
| | B: 10 mM NH4Ac (pH=8.5):ACN = 2:8 (v/v) | |
| Elution gradient | Time (min) | %B |
| | 0.0 | 10 |
| | 7.0 | 90 |
| | 8.0 | 90 |
| | 8.1 | 20 |
| | 11.0 | 20 |
| Running time | 11.0 min | |
| Post-run time | 0.0 min | |
| Mobile phase flow rate | 1.0 mL/min | |
| Injection volume | 5 µL | |
| Detection wavelength | 270 nm | |
| Column temperature | 40 °C | |
| Injector temperature | Room temperature | |

Instruments and methods known in this field can be used for instruments and methods that are not clearly indicated in the "instruments and methods" section.

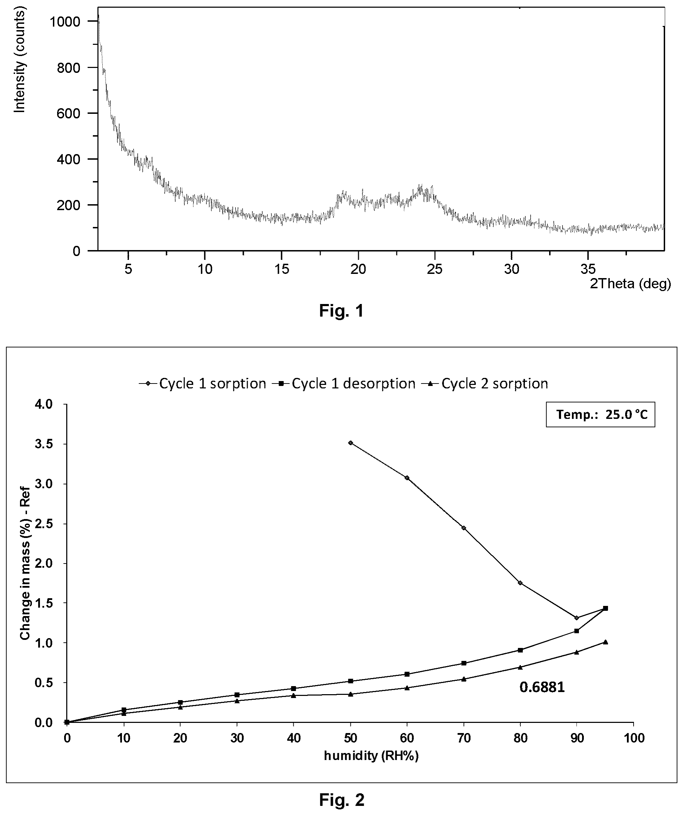

Example 1a (compound 1 amorphous, repeated Chinese patent application No. 201210190520.4 example 1): according to the method of Chinese patent application No. 201210190520.4 example 1, the crude product obtained in step 13 of example 1 was separated and purified by silica gel column chromatography, with dichloromethane/methanol as eluent, and then evaporated to obtain a white solid, which was amorphous by XRPD analysis, and its XRPD diagram was shown in Fig. 1, and dynamic water adsorption (DVS) as shown in Fig. 2.Example 2a: adding 4 mL anhydrous ethanol to the amorphous sample of about 21 mg example 1a, heating and stirring in an oil bath at 85 ° C for 5 minutes to dissolve the solution; then, the obtained clear liquid was cooled to 25 ° C within 2.5 hours with stirring at room temperature, and the solid sample was filtered out for XRPD determination. The XRPD results were shown in Fig. 3. The TGA/DSC results (Fig. 4) showed that the sample was heated to 150 °C with a weight loss of 0.77%, and there was a sharp endothermic peak at 221.3 °C (initial temperature). ¹H NMR data were collected with DMSO-d6 as solvent, and no obvious solvent residue was detected as shown in Fig. 5. According to the characterization results of crystalline form I, it had a small and gentle weight loss (less than 1.80% of the theoretical water content of hemihydrate) and a single melting endothermic signal, so it was inferred that crystalline form I is hydrate-free or crystal-free. The XRPD diffraction peak data of the crystalline form I were shown in Table 6.

**Table 6: XRPD diffraction peak data of crystalline form I.**

| #Peak | 2θ [°] | Interplanar crystal spacing [Å] | Rel. Int. [%] |
|---|---|---|---|
| 1 | 5.30 | 16.69 | 30.40 |
| 2 | 7.48 | 11.82 | 44.34 |
| 3 | 11.81 | 7.50 | 29.26 |
| 4 | 14.85 | 5.97 | 4.25 |
| 5 | 15.83 | 5.60 | 28.43 |
| 6 | 16.71 | 5.31 | 62.99 |
| 7 | 17.92 | 4.95 | 29.09 |
| 8 | 18.95 | 4.68 | 26.30 |
| 9 | 19.17 | 4.63 | 28.46 |
| 10 | 19.43 | 4.57 | 24.01 |
| 11 | 21.14 | 4.20 | 19.40 |
| 12 | 21.82 | 4.07 | 55.69 |
| 13 | 22.36 | 3.98 | 35.44 |
| 14 | 23.75 | 3.75 | 100.00 |
| 15 | 24.57 | 3.62 | 30.67 |
| 16 | 27.08 | 3.29 | 35.05 |
| 17 | 27.83 | 3.21 | 22.35 |
| 18 | 28.88 | 3.09 | 13.36 |
| 19 | 31.20 | 2.87 | 12.50 |
| 20 | 31.92 | 2.80 | 20.15 |
| 21 | 32.40 | 2.76 | 24.34 |
| 22 | 33.91 | 2.64 | 12.43 |
| 23 | 35.83 | 2.51 | 2.49 |
| 24 | 37.51 | 2.40 | 9.02 |
| 25 | 39.04 | 2.31 | 6.06 |

Example 2b: the amorphous sample of about 20 mg each was added to a 20 mL vial, dissolved in a good solvent of 0.4 mL (see Table 7), and filtered (a PTFE filter membrane with a pore diameter of 0.45 µm) to obtain a clarified solution. While stirring the clarified solution, the antisolvent in Table 7 was added until the solid was precipitated, and the solid was separated by centrifugation and XRPD test. As shown in Table 7, the solid was obtained by adding antisolvent, and the crystalline form I was determined by XRPD.

**Table 7: summary of anti-solvent addition test**

| Examples | Weight (mg) | Solvents | Antisolvents | Results |
|---|---|---|---|---|
| Example 2b1[2] | 20.6 | MEK (each 1 mL) | MTBE (5 mL) | Crystalline form I |
| Example 2b2[3] | 19.9 | | EtOAc (5 mL) | Crystalline form I |
| Example 2b3[3] | 20.1 | | CHCl3 (5 mL) | Crystalline form I |
| Example 2b4 | 20.8 | | n-Heptane (3 mL) | Crystalline form I |
| Example 2b5 | 20.6 | 1,4-Dioxane (each 1 mL) | Anisole (5 mL) | Crystalline form I |
| Example 2b6[1] | 20.4 | | EtOAc (5 mL) | Crystalline form I |
| Example 2b7 | 20.8 | | n-Heptane (5 mL) | Crystalline form I |
| Example 2b8 | 20.5 | | _{H2O} (2 mL) | Crystalline form I |
| Example 2b9[3] | 19.7 | DMSO (each 0.4 mL) | IPAc (5 mL) | Crystalline form I |
| Example 2b10[3] | 20 | | CPME (5 mL) | Crystalline form I |
| Example 2b11[3] | 19.6 | | DCM (5 mL) | Crystalline form I |
| Example 2b12[4] | 19.6 | | Toluene (5 mL) | Crystalline form I |

| | | | | |
|---|---|---|---|---|
| ^{[1]}: Clarify after adding anti solvent, turned to 5 °C and stirred to precipitate solids. ^{[2]}: Clarify after adding antisolvent, turned to 5 °C and still clarify after stirring, and turned to - 20 °C to stir precipitated solid. ^{[3]}: Clarify after adding antisolvent, turned to 5 °C and -20 °C stirring and still clarify, turned to room temperature volatilization. ^{[4]}: After adding the antisolvent, it became oil. After circulating and stirring at a temperature of 5-50 °C, it still became oil and evaporated at room temperature. | | | | |

Example 2c: weigh the amorphous sample of example 1a of about 20 mg each in a 5 mL or HPLC vial, add the solvent in Table 8 of 0.7-4.0 mL, stirred at 50 °C for about 2 hours, then filtered the filtrate (a PTFE membrane with a pore size of 0.45 µm), placed the filtrate in a biochemical incubator, cooled it to 5 °C at a cooling rate of 0.1 °C/min, collected the precipitated solids and performed XRPD testing. The test results were shown in Table 8. The solid was obtained by slow cooling test and was determined as crystalline form I by XRPD.

**Table 8 slow cooling test summary at 5 °C**

| Examples | Weight | Solvents | Volume (mL) | Results |
|---|---|---|---|---|
| Example 2c1^{[2]} | 19.9 | MIBK | 1.0 | Crystalline form I |
| Example 2c2 | 20.7 | Methyl acetate | 1.0 | Crystalline form I |
| Example 2c3^{[2]} | 20.4 | 2-MeTHF | 0.7 | Crystalline form I |
| Example 2c4^{[1]} | 20.5 | Acetone/EtOH (1:1) | 0.7 | Crystalline form I |

| | | | | |
|---|---|---|---|---|
| ^{[1]}: Slowly cooled to 5 °C and clear, then transferred to -20 °C and let stand before releasing the solid. ^{[2]}: Slowly reduced the temperature to 5 °C and clarify, transferred to -20 °C, there was still no solid precipitation after static setting, and transferred to room temperature volatilization. | | | | |

### Example 3 Crystalline form II

Example 3a: Weighed 71.6 mg of crystalline form I sample of Example 2a into a 3 mL vial, added 1.5 mL of ACN, and stirred magnetically at room temperature for about 1 day. Centrifuged the solid and dried it under environmental conditions (~21 °C/45% RH) for about 4 hours to obtain the final product, which was then subjected to XRPD and TGA/DSC testing. The XRPD results were shown in Figure 6. The TGA results were shown in Figure 7, TGA showed that heating the sample to 120 °C resulted in 5.90% stepwise weight loss; The DSC results indicated that there were two endothermic peaks at 94.1 °C and 220.9 °C (starting temperature), and one exothermic peak at 191.6 °C (peak temperature). The¹H NMR results were shown in Figure 8, the molar ratio of ACN to API in the sample was 0.8 (5.9 weight%, consistent with the weight loss of TGA). Based on the characterization and heating experimental results, it was speculated that crystalline form II was an ACN solvent complex, and after heating, the desolvation underwent crystal transformation. The XRPD diffraction peak data of this crystalline form II could be found in Table 9.

**Table 9: XRPD diffraction peak data of crystalline form II**

| #peak | 2θ [°] | Interplanar crystal spacing [Å] | Rel. Int. [%] |
|---|---|---|---|
| 1 | 6.35 | 13.90 | 50.21 |
| 2 | 9.18 | 9.63 | 34.38 |
| 3 | 9.91 | 8.92 | 10.86 |
| 4 | 14.44 | 6.13 | 25.09 |
| 5 | 16.03 | 5.52 | 42.80 |
| 6 | 18.25 | 4.86 | 33.79 |
| 7 | 19.77 | 4.49 | 10.15 |
| 8 | 20.34 | 4.36 | 69.45 |
| 9 | 21.81 | 4.07 | 14.52 |
| 10 | 22.41 | 3.96 | 74.27 |
| 11 | 22.60 | 3.93 | 81.90 |
| 12 | 23.84 | 3.73 | 10.10 |
| 13 | 24.64 | 3.61 | 19.76 |
| 14 | 24.99 | 3.56 | 100.00 |
| 15 | 25.43 | 3.50 | 6.69 |
| 16 | 26.05 | 3.42 | 94.96 |
| 17 | 26.41 | 3.37 | 23.03 |
| 18 | 27.07 | 3.29 | 48.38 |
| 19 | 28.71 | 3.11 | 57.10 |
| 20 | 29.08 | 3.07 | 28.99 |
| 21 | 29.81 | 3.00 | 8.41 |
| 22 | 31.16 | 2.87 | 4.91 |
| 23 | 31.57 | 2.83 | 7.74 |
| 24 | 32.27 | 2.77 | 27.92 |
| 25 | 32.68 | 2.74 | 25.25 |
| 26 | 33.93 | 2.64 | 35.81 |
| 27 | 34.19 | 2.62 | 9.92 |
| 28 | 35.42 | 2.53 | 10.46 |
| 29 | 37.07 | 2.42 | 26.08 |
| 30 | 37.56 | 2.39 | 11.92 |
| 31 | 38.69 | 2.33 | 4.55 |
| 32 | 39.51 | 2.28 | 16.48 |

Example 3b: Weigh 20.4 mg of crystalline form I sample from Example 2a into a 3 mL vial, add 2 mL of ACN/acetone (1:1, v/v), sonicate and filter (0.45µm PTFE filter membrane) to obtain a clear solution. Seal with sealing film and tie 4 small holes, placed in a fume hood and slowly evaporate for 5 days. Solid precipitation was observed and left under environmental conditions overnight, then transferred to room temperature and vacuum dried for about 1 day. Slowly evaporate the ACN/acetone (1:1, v/v) solution of crystalline form I of Example 2a, precipitate the solid, and then transfer it to room temperature vacuum drying to obtain the final product, which is then subjected to XRPD testing. The XRPD results are shown in Figure 6.

Example 3c: Weigh approximately 19.7 mg of the amorphous sample obtained from Example 1a into an HPLC vial, add 0.5 mL of ACN/_{H2O} (19: 1) solvent, and obtain a suspension. Place the suspension at 5 °C with magnetic stirring (~1000 rpm) for about 6 days. Centrifuge the solid and perform XRPD testing. The XRPD result is crystalline form II.

Example 3d: Weigh approximately 19.9 mg of the amorphous sample obtained from Example 1a into an HPLC vial, add 0.5 mL of ACN/acetone (1:1) solvent, and obtain a suspension. Place the suspension at 5 °C and stir magnetically (~1000 rpm) for about 6 days. Centrifuge the solid and perform XRPD testing. The XRPD result is crystalline form II.

### Example 4 (crystalline form IIIA and IIIB)

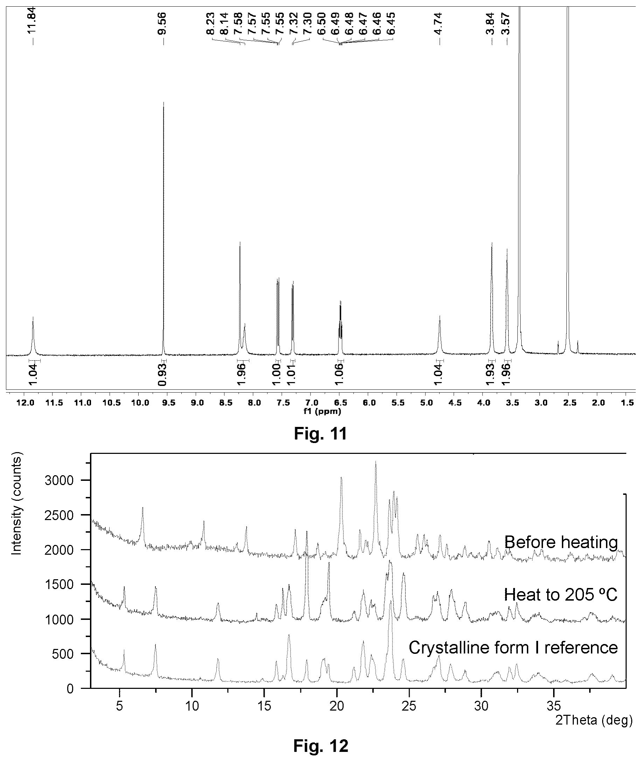

Example 4a: Heat the crystalline form II sample of Example 3a with DSC to 120 °C and maintain a constant temperature for 5 minutes before cooling to room temperature to obtain crystalline form IIIA. The XRPD results are shown in Figure 9. The TGA/DSC results (Fig. 10) show that the sample loses 0.26% weight when heated to 200 °C, which is lower than the theoretical water content of the semi hydrate (1.80%). The DSC results show that there is one exothermic signal at 200.3 °C (peak temperature) and one sharp endothermic signal at 219.6 °C (starting temperature). The 1H NMR results (Fig. 11) showed that no significant solvent residue was detected. Heat the crystalline form IIIA to 205 °C, cool it to room temperature, and expose it to environmental conditions before transforming into crystalline form I. The XRPD results before and after heating are shown in Figure 12. Based on the characterization results of crystalline form IIIA, it is speculated that crystalline form IIIA is hydrate free, and the exothermic signal at 198.8 °C is a thermal signal for the transition to crystalline form I. The XRPD diffraction peak data of this crystalline form IIIA can be found in Table 10.

**Table 10 XRPD diffraction peak data of crystalline form IIIA**

| #peak | 2θ [°] | Interplanar crystal spacing [Å] | Rel. Int. [%] |
|---|---|---|---|
| 1 | 6.59 | 13.41 | 38.94 |
| 2 | 9.90 | 8.93 | 6.81 |
| 3 | 10.80 | 8.19 | 31.28 |
| 4 | 13.09 | 6.76 | 8.78 |
| 5 | 13.75 | 6.44 | 28.04 |
| 6 | 17.14 | 5.17 | 28.86 |
| 7 | 17.87 | 4.96 | 3.27 |
| 8 | 18.71 | 4.74 | 13.40 |
| 9 | 19.19 | 4.63 | 4.40 |
| 10 | 20.32 | 4.37 | 80.68 |
| 11 | 21.59 | 4.12 | 27.30 |
| 12 | 21.97 | 4.05 | 17.85 |
| 13 | 22.69 | 3.92 | 100.00 |
| 14 | 23.62 | 3.77 | 59.78 |
| 15 | 23.91 | 3.72 | 68.84 |
| 16 | 24.15 | 3.69 | 63.99 |
| 17 | 25.54 | 3.49 | 23.09 |
| 18 | 26.01 | 3.43 | 23.93 |
| 19 | 27.13 | 3.29 | 23.69 |
| 20 | 27.59 | 3.23 | 14.30 |
| 21 | 28.83 | 3.10 | 12.47 |
| 22 | 29.24 | 3.05 | 5.86 |
| 23 | 30.51 | 2.93 | 18.64 |
| 24 | 31.13 | 2.87 | 9.85 |
| 25 | 31.79 | 2.82 | 6.52 |
| 26 | 33.60 | 2.67 | 6.98 |
| 27 | 34.14 | 2.63 | 11.49 |
| 28 | 36.08 | 2.49 | 6.97 |
| 29 | 36.67 | 2.45 | 2.54 |
| 30 | 37.26 | 2.41 | 5.82 |

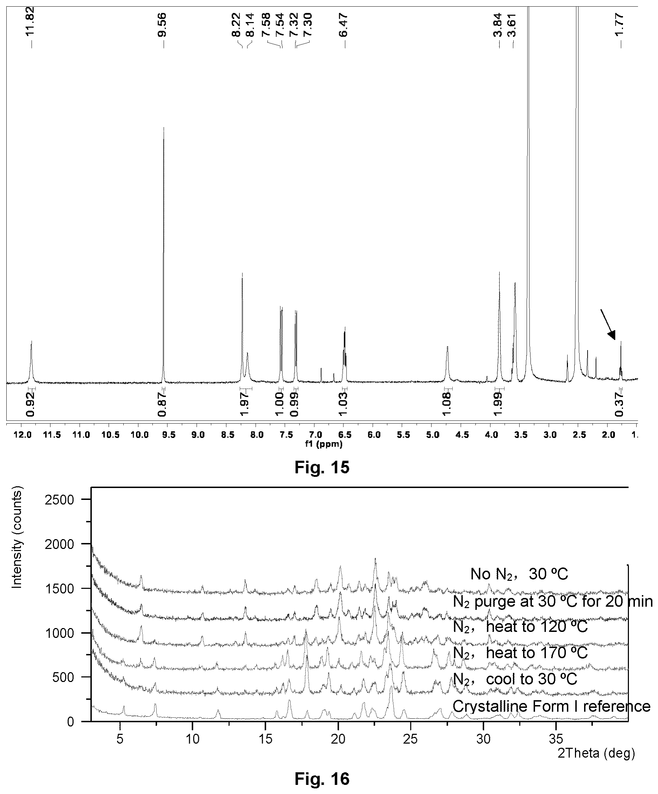

Example 4b: Weigh 100.0 mg of crystalline form I sample from Example 2a into a 20 mL vial, add 10 mL of THF to dissolve, filter (0.45 µm PTFE filter membrane) to obtain a clear solution. The filtrate is treated with rotary evaporation at 50 °C, and the final product was collected. The XRPD results are shown in Figure 13. The TGA/DSC results (Fig. 14) showed that the sample lost 3.49% weight when heated to 200 °C. The DSC results show that there is a weak endothermic signal and an exothermic signal at 121.3 °C and 144.6 °C (peak temperature), respectively, and a strong endothermic signal at 218.3 °C (starting temperature). The ¹H NMR results (Fig. 15) show that the molar ratio of residual solvent THF to API is 0.09 (1.3 weight%), suggesting that it is a solvent adsorbed on the surface. The thermal signals of crystalline form IIIB at 121.3 °C and 144.6 °C are the thermal signals for the transition to crystalline form I. The XRPD diffraction peak data of this crystalline form IIIB can be found in Table 11.

**Table 11: XRPD diffraction peak data of crystalline form IIIB**

| #peak | 2θ [°] | Interplanar crystal spacing [Å] | Rel. Int. [%] |
|---|---|---|---|
| 1 | 6.53 | 13.52 | 25.02 |
| 2 | 10.75 | 8.23 | 15.71 |
| 3 | 12.62 | 7.01 | 8.28 |
| 4 | 13.05 | 6.78 | 14.77 |
| 5 | 13.69 | 6.46 | 40.38 |
| 6 | 16.63 | 5.33 | 11.35 |
| 7 | 17.07 | 5.19 | 19.30 |
| 8 | 18.60 | 4.77 | 27.70 |
| 9 | 19.59 | 4.53 | 8.59 |
| 10 | 20.19 | 4.39 | 58.98 |
| 11 | 20.82 | 4.26 | 21.02 |
| 12 | 21.52 | 4.13 | 35.89 |
| 13 | 21.93 | 4.05 | 36.74 |
| 14 | 22.64 | 3.92 | 100.00 |
| 15 | 23.57 | 3.77 | 52.72 |
| 16 | 24.01 | 3.70 | 26.55 |
| 17 | 25.46 | 3.50 | 8.40 |
| 18 | 26.13 | 3.41 | 37.54 |
| 19 | 27.55 | 3.24 | 28.62 |
| 20 | 30.46 | 2.93 | 43.71 |
| 21 | 31.04 | 2.88 | 8.06 |
| 22 | 31.79 | 2.81 | 18.05 |
| 23 | 32.81 | 2.73 | 6.62 |
| 24 | 33.54 | 2.67 | 5.67 |
| 25 | 34.06 | 2.63 | 8.44 |
| 26 | 34.46 | 2.60 | 8.74 |

### Example 5 (crystalline form IV)

Example 5a: Weigh approximately 20 mg of the amorphous sample obtained from Example 1a into an HPLC vial, add 0.5 mL1,4-dioxane solvent, and place the obtained suspension at room temperature with magnetic stirring (~1000 rpm) for about 6 days. Centrifuge the solid and dry it overnight under vacuum at room temperature to obtain the final product, which is then subjected to XRPD and TGA/DSC testing. The XRPD and TGA/DSC results are shown in Fig 17 and Figure 18. The TGA results showed that 16.80% of the samples lost weight in a stepped manner when heated to 120 °C. The DSC results indicate that there are two endothermic peaks at 95.6 °C and 220.5 °C (starting temperature). The¹H NMR results are shown in Figure 19, the molar ratio of 1,4-dioxane to API in the sample is 1.2 (17.8 weight%, consistent with TGA weight loss). Based on the above characterization results and variable temperature XRPD results, it is speculated that crystalline form IV is a 1,4-dioxane solvate. The XRPD diffraction peak data of this crystalline form IV are shown in Table 12.

**Table 12: XRPD diffraction peak data of crystalline form IV**

| #peak | 2θ [°] | Interplanar crystal spacing [Å] | Rel. Int. [%] |
|---|---|---|---|
| 1 | 5.26 | 16.79 | 6.24 |
| 2 | 8.56 | 10.33 | 12.76 |
| 3 | 9.85 | 8.98 | 4.26 |
| 4 | 13.29 | 6.66 | 35.90 |
| 5 | 17.69 | 5.01 | 23.16 |
| 6 | 18.29 | 4.85 | 6.66 |
| 7 | 19.75 | 4.49 | 24.35 |
| 8 | 20.35 | 4.36 | 5.79 |
| 9 | 21.09 | 4.21 | 6.80 |
| 10 | 22.45 | 3.96 | 100.00 |
| 11 | 22.86 | 3.89 | 21.15 |
| 12 | 23.44 | 3.80 | 12.39 |
| 13 | 24.44 | 3.64 | 1.79 |
| 14 | 25.68 | 3.47 | 21.54 |
| 15 | 26.57 | 3.35 | 9.10 |
| 16 | 27.52 | 3.24 | 8.17 |
| 17 | 28.40 | 3.14 | 1.61 |
| 18 | 29.78 | 3.00 | 3.82 |
| 19 | 31.43 | 2.85 | 5.30 |
| 20 | 31.83 | 2.81 | 23.10 |
| 21 | 32.81 | 2.73 | 17.25 |
| 22 | 34.29 | 2.62 | 4.72 |
| 23 | 35.69 | 2.52 | 7.27 |
| 24 | 37.72 | 2.38 | 5.24 |

Example 5b: The crystalline form I sample (500 mg) of Example 2a was stirred at room temperature in 1,4-dioxane (12.5 mL) for 2 days, and the resulting solid was vacuum dried at room temperature for 1 day. The XRPD results are shown in Fig 17.

### Example 6 (Crystalline form V)

Example 6a: Weigh 20.8 mg of crystalline form I sample from Example 2a into a 5 mL vial and add 4 mL of EtOH. Stir at 70 °C and dissolve, then cool within 650 minutes (cooling rate of 0.1 °C /min) to 5 °C and let it stand at 5 °C. After removing the solution, leave the solid open and dry under environmental conditions (temperature: ~21 °C, humidity: ~36% RH). The XRPD results are shown in Fig 20. The TGA/DSC results (Fig. 21) show that there is a 7.64% stepwise weight loss when the sample was heated to 120 °C, and there were two endothermic peaks at 103.1 °C and 223.3 °C (starting temperature). The ¹H NMR results are shown in Figure 22, the molar ratio of EtOH to API in the sample is 0.7 (6.2 weight%, consistent with the weight loss of TGA). Based on the above characterization results and variable temperature XRPD results, it is speculated that the crystalline form V is an EtOH solvate, which undergoes desolvation and transformation into anhydrous crystalline form I after heating. The XRPD diffraction peak data of the crystalline form V are shown in Table 13.

**Table 13: XRPD diffraction peak data of crystalline form V**

| #peak | 2θ [°] | Interplanar crystal spacing [Å] | Rel. Int. [%] |
|---|---|---|---|
| 1 | 6.21 | 14.23 | 100.00 |
| 2 | 8.47 | 10.44 | 82.35 |
| 3 | 9.06 | 9.76 | 17.23 |
| 4 | 9.61 | 9.21 | 23.79 |
| 5 | 12.42 | 7.13 | 2.06 |
| 6 | 14.26 | 6.21 | 8.59 |
| 7 | 15.62 | 5.67 | 63.47 |
| 8 | 16.95 | 5.23 | 5.59 |
| 9 | 17.55 | 5.05 | 50.65 |
| 10 | 19.25 | 4.61 | 30.15 |
| 11 | 20.07 | 4.43 | 10.07 |
| 12 | 21.73 | 4.09 | 52.92 |
| 13 | 22.22 | 4.00 | 30.54 |
| 14 | 23.12 | 3.85 | 35.57 |
| 15 | 24.32 | 3.66 | 7.34 |
| 16 | 24.94 | 3.57 | 2.94 |
| 17 | 25.53 | 3.49 | 56.04 |
| 18 | 25.94 | 3.43 | 67.57 |
| 19 | 28.05 | 3.18 | 55.07 |
| 20 | 28.49 | 3.13 | 13.01 |
| 21 | 30.21 | 2.96 | 11.66 |
| 22 | 31.25 | 2.86 | 13.34 |
| 23 | 32.33 | 2.77 | 4.66 |
| 24 | 32.92 | 2.72 | 36.12 |
| 25 | 34.22 | 2.62 | 26.83 |
| 26 | 35.47 | 2.53 | 13.10 |
| 27 | 36.43 | 2.47 | 3.79 |
| 28 | 37.86 | 2.38 | 1.95 |
| 29 | 38.94 | 2.31 | 14.63 |
| 30 | 39.78 | 2.27 | 9.04 |

### Example 7 (Crystalline form VI)

Dissolve the amorphous sample (1 g) of Example 1a in DMF (3 mL). After complete dissolving, add IPA (6 mL), stir overnight at room temperature. Precipitate the solid, filter, wash the filter cake with isopropanol. Drying under vacuum give the solvate crystalline form of DMF, which is crystalline form VI.

The XRPD results of crystalline form VI are shown in Figure 23. DSC results show that there are two endothermic peaks at 147.39 ° C and 208.56 ° C. The TGA results of Fig. 24 show a weight loss of 0.67% when heated to 180 ° C.

### Example 8 (Crystal Conversion)

Example 8a: The crystalline form IV in Example 5a was heated to 120 °C, cooled to room temperature, and exposed to environmental conditions before transforming into crystalline form I. The XRPD results are shown in Fig As shown in 25.

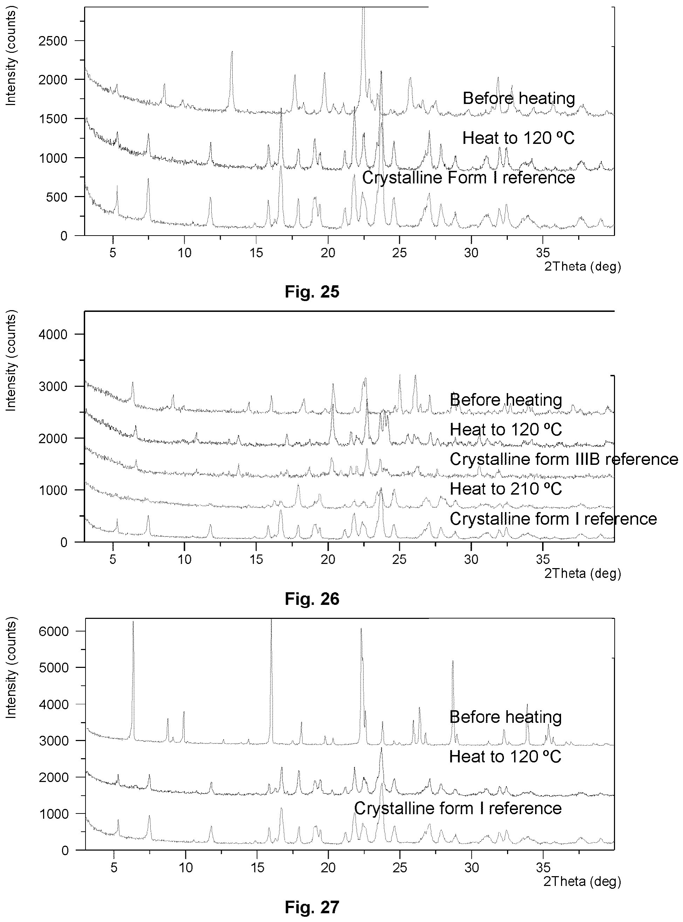

Example 8b: Heat the crystalline form II of Example 3a to 120 °C and 210 °C, cool it to room temperature, and expose it to environmental conditions. The XRPD results are shown in Fig 26 shows that when heated to 120 °C, it transforms into crystalline form IIIB, and when heated to 210 °C, it transforms into crystalline form I.

Example 8c: The crystalline form II of Example 3b was heated to 120 °C, cooled to room temperature, and exposed to environmental conditions before transforming into crystalline form I. The XRPD results are shown in Fig 27. It is speculated that the sample may also first transform into crystalline form IIIB after heating, but it quickly transforms into crystalline form I under environmental conditions, which was shown to transform into crystalline form I during XRPD testing.

Example 8d: Identification of crystalline form IIIB in Example 4a by variable temperature XRPD, as shown in Fig As shown in Figure 16. After 20 minutes of N2 blowing at 30 °C, the crystal structure remained unchanged. When heated to 120 °C under N2 protection, a diffraction peak of crystal structure I was observed. When further heated to 170 °C, the main diffraction peak was crystal structure I, with only a small amount of diffraction peaks of crystal structure IIIB. When cooled to 30 °C under N₂ protection, it did not continue to transform.

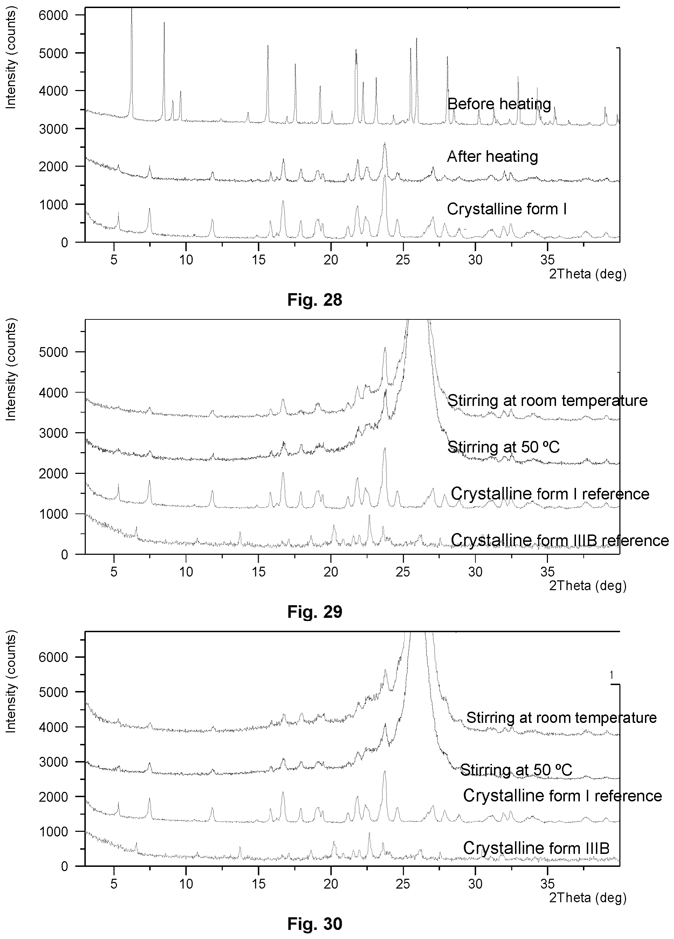

Example 8e: The crystalline form V of Example 6a was heated to 120 °C, cooled to room temperature, and exposed to environmental conditions before transforming into crystalline form I. The XRPD results are shown in Fig As shown in Figure 28.

### Example 8f (competitive suspension experiment for crystalline forms I and IIIA)

This embodiment involves slurry competition experiments of crystalline forms I and IIIA in EtOAc and MIBK at room temperature and 50 °C, as well as slurry competition experiments in acetone/_{H2O} with different water activities at room temperature.

Weigh approximately 15 mg of crystalline form I sample from Example 2a and add 1 mL of the corresponding solvent to the HPLC bottle. Stir at the corresponding temperature for 4 hours or overnight. Filter (0.45 µ Obtain saturated solution using PTFE filter membrane. Weigh the samples of crystalline form I and IIIA in Example 2 and Example 4a of equal mass (approximately 5 mg each) into new HPLC bottles, and add the saturated solution obtained in Step 1. Stir at the corresponding temperature and perform XRPD testing on the solid wet sample (covered with Kapton film to avoid potential crystal transformation caused by solvent evaporation during the testing process).

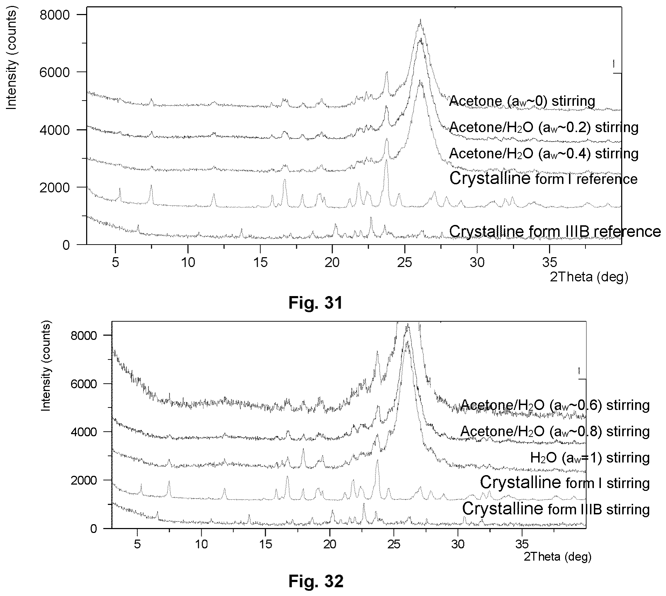

The results of slurry competition are summarized in Table 14. The XRPD results are shown in Fig 29. Fig 30. Fig 31. Fig 32.

**Table 14 Results of Slurry competition Experiment for Crystalline forms I and IIIA**

| Experiment ID | Initial crystal form | Solvent (v/v) | Temperature | Result |
|---|---|---|---|---|
| Example 8f1 | crystalline | EtOAc | Room temperature | Crystalline form I |
| Example 8f2 | form I and IIIA | EtOAc | 50 °C | Crystalline form I |
| Example 8f3 | | MIBK | Room temperature | Crystalline form I |
| Example 8f4 | | MIBK | 50 °C | Crystalline form I |
| Example 8f5 | | acetone (aw~0) | Room temperature | Crystalline form I |
| Example 8f6 | | acetone/_{H2O} (aw~0.2, 984:16) | Room temperature | Crystalline form I |
| Example 8f7 | | acetone/_{H2O} (aw~0.4, 952:48) | Room temperature | Crystalline form I |
| Example 8f8 | | acetone/_{H2O} (aw~0.6, 857:143) | Room temperature | Crystalline form I |
| Example 8f9 | | acetone/_{H2O} (aw~0.8, 604:396) | Room temperature | Crystalline form I |
| Example 8f10 | | H₂O (aw=1) | Room temperature | Crystalline form I |

Where aw: Theoretical water activity.

According to the XRPD comparison results, the physical mixture of crystalline forms I and IIIA transformed into crystalline form I after slurry competition under all conditions, indicating that under anhydrous conditions in the range of room temperature to 50 °C and water activity conditions of 0-1 at room temperature, anhydrous crystalline form I is thermodynamically more stable than crystalline form IIIA.

### Example 9 (hygroscopicity of crystalline form I)

The hygroscopicity of crystalline form IIIA in Example 2a, amorphous in Example 1a, and crystalline form IIIA in Example 4a was evaluated through dynamic water adsorption (DVS) tests at 25 °C.

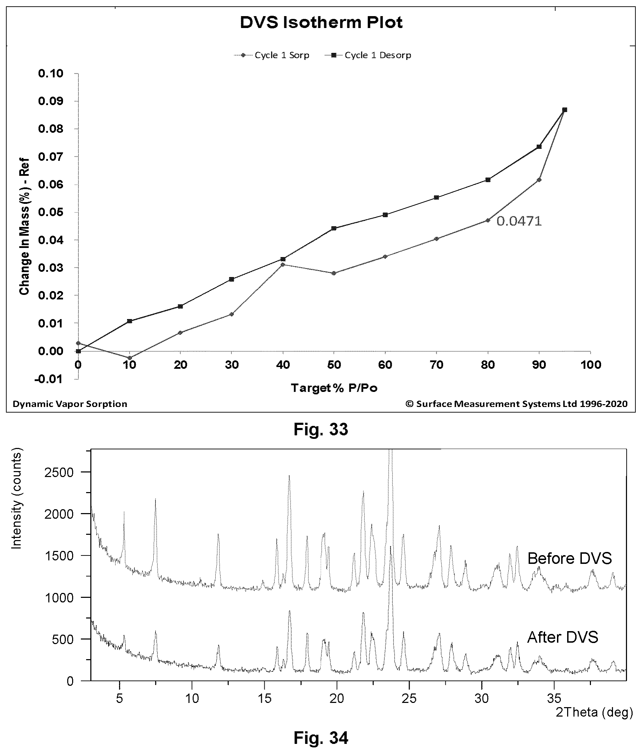

The DVS results of crystalline form I are shown in Fig As shown in Figure 33, the water absorption and weight gain at 25 °C/80% RH are 0.047%, indicating almost no hygroscopicity. The XRPD comparison results (Fig. 34) show that crystalline form I did not undergo crystalline form transformation after DVS testing.

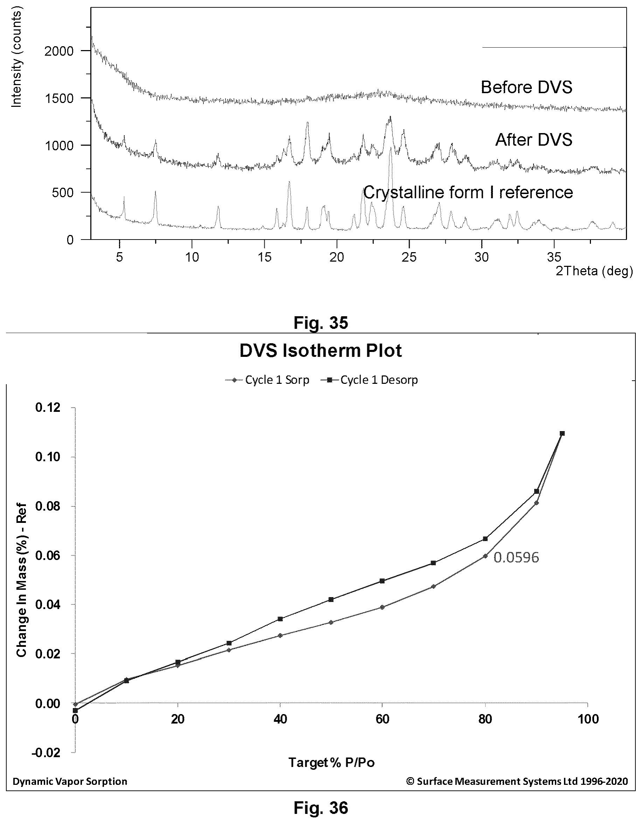

The amorphous DVS results are shown in Fig As shown in Figure 2, as the humidity increases from 50% RH to 95% RH, the water absorption and weight gain continuously decrease. It is speculated that amorphous samples undergo crystal transformation during the humidity increase process, and the water or organic solvents adsorbed or encapsulated in the original sample are removed by N₂ blowing. The XRPD comparison results (Fig. 35) show that the amorphous sample transformed into crystalline form I after DVS testing.

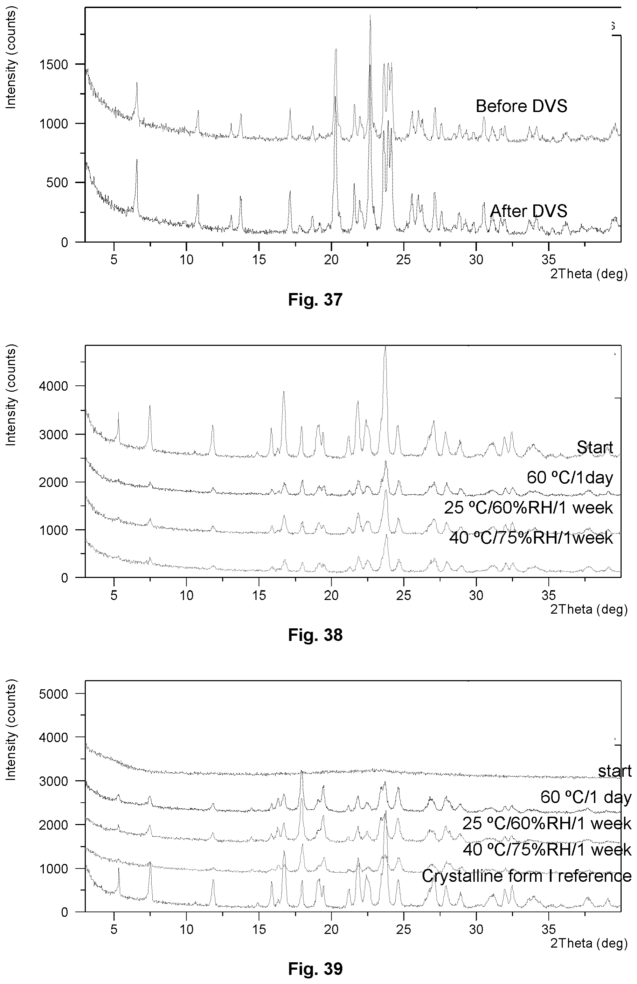

The DVS results of crystalline form IIIA are shown in Fig As shown in Figure 36, the water absorption and weight gain at 25 °C/80% RH are 0.060%, indicating almost no hygroscopicity. The XRPD comparison results (Fig. 37) show that crystalline form IIIB did not undergo crystalline form transformation after DVS testing.

### Example 10 (Solid state stability of crystalline form I)

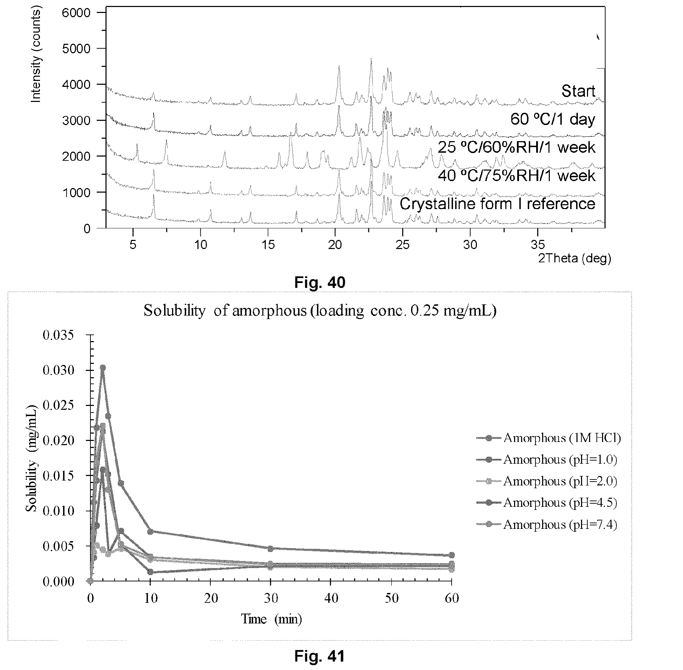

In order to evaluate the solid-state stability of crystalline forms I, IIIA, and amorphous, appropriate amounts of amorphous samples from Example 2a, Example 1a, and Example 4a were weighed, and stability experiments were conducted under conditions of 60 °C/closed/1 day, 25 °C/60% RH/open/1 week, and 40 °C/75% RH/open/1 week, respectively. Evaluate the physical stability of stable samples under different conditions by XRPD testing for crystalline form, and evaluate the chemical stability by HPLC testing for purity. The evaluation results are summarized in Table 15, and the XRPD results are shown in Fig 38. Fig 39 and Fig 40 (A weak diffraction peak of crystalline form I was observed at the star mark in Fig. 40). The stability results showed that crystalline form I did not undergo any crystalline form transformation or purity reduction under the three evaluation conditions, indicating that crystalline form I has good physical and chemical stability under the evaluation conditions; The purity of amorphous materials did not show significant changes under three evaluation conditions, but they all transformed into crystalline form I; A weak diffraction peak of crystalline form I was observed in crystalline form IIIA after being left open for 1 week at 60 °C, while the crystalline form remained unchanged after being left open for 1 week at 25 °C/60% RH and 40 °C/75% RH.

**Table 15 Summary of Solid State Stability Evaluation Results for Crystalline form I in Example 2a**

| Initial crystal form | Condition | Purity(area%) | Crystalline form change |
|---|---|---|---|
| Crystalline form I of example 2a | Initial | 100.00 | -- |
| | 60 °C/sealed/1day | 100.00 | No |
| | 25 °C/60%RH/open/1week | 100.00 | No |
| | 40 °C/75%RH/open/1week | 100.00 | No |
| Amorphous of example 1a | Initial | 100.00 | -- |
| | 60 °C/sealed/1 day | 100.00 | Yes* |
| | 25 °C/60%RH/open/1 week | 100.00 | Yes* |
| | 40 °C/75%RH/open/1 week | 100.00 | Yes* |
| Crystalline form IIIA | Initial | 100.00 | -- |
| | 60 °C/sealed/1day | 100.00 | Yes# |
| | 25 °C/60%RH/open/1 week | 100.00 | No |
| | 40 °C/75%RH/open/1 week | 100.00 | No |

| | | | |
|---|---|---|---|
| *: The amorphous samples all transformed into crystalline form I after stability evaluation; #: weak diffraction peaks of crystalline form I were observed | | | |

### Example 11 (solubility)

The dynamic solubility of crystalline form I in Example 2a and amorphous in Example 1a under different pH conditions (1 M HCl and pH 2.0/4.5/6.8/7.4 buffer) at room temperature was tested. The specific steps are as follows:
(1) Weigh about 2.5 mg of crystalline form I or amorphous sample into a 20 mL glass bottle and add 10 mL buffer of different pH, or weigh about 2.0 mg of crystalline form I or amorphous sample into a 20 mL glass bottle and add 8 mL hydrochloric acid (1 M).
(2) After shaking (~500 rpm) for 3min at room temperature, 0.8-1 mL samples were sucked by a syringe, filtered (0.45 µm PTFE filter membrane) and tested by HPLC.
(3) For the solution obtained from amorphous material, remove 100 µL and dilute 10 times with the corresponding buffer for later use. If there is solid precipitation in the diluted solution, the diluted sample will be tested. If there is no solid precipitation, the diluted sample will be tested. For the solution obtained from crystalline form I, which was expected to be of low solubility, no dilution was performed and the filtered clear solution was directly tested by HPLC.

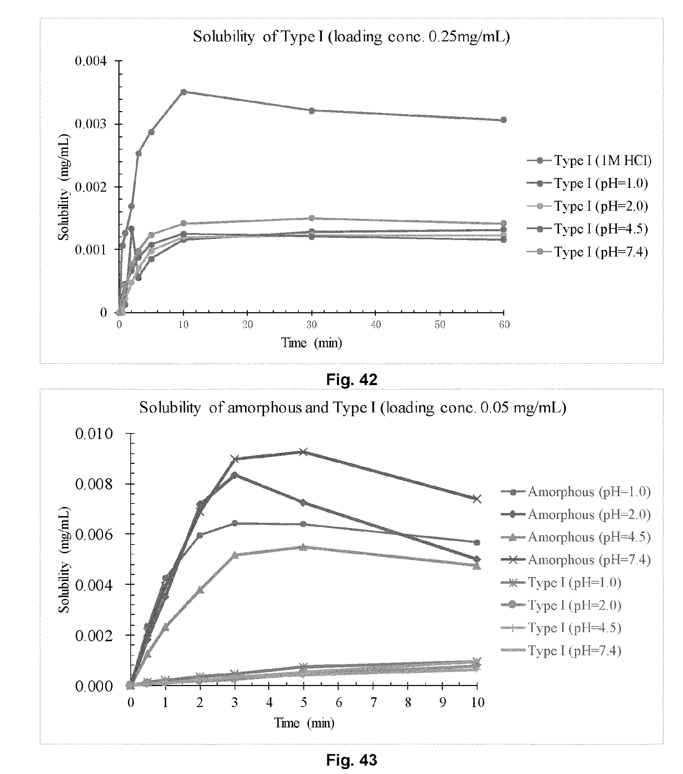

The solubility of the crystalline form I and the amorphous form at different PH conditions is shown in Table 16. The results showed that the solubility of crystalline form I almost did not change at different pH, while the amorphous form showed a large change with the change of pH (Fig. 41). However, the dissolution of genotype I in vivo is more stable (as shown in Fig. 42), and it is easier to obtain the pharmacokinetic results in vivo with stable blood concentration, which is beneficial to avoid the risk of drug safety caused by too large fluctuations in blood concentration. Similar results were observed when the feed concentration was 0.05 mg/mL (e.g. Fig. 43).

**Table 16 3min solubility results of crystalline form I and amorphous form in different media (feed concentration at 0.25 mg/mL)**

| | solubility(mg/mL) | |
|---|---|---|
| Media | Amorphous form | Crystalline Form I |
| 1 M HCl | 0.023 | 0.0025* |
| pH 1.0 | 0.0039 | 0.0006 |
| pH 2.0 | 0.0039 | 0.0007 |
| pH 4.5 | 0.015 | 0.0009 |
| pH 7.4 | 0.013 | 0.0010 |

| | | |
|---|---|---|
| LOQ=0.28 µg/mL_{∘} | | |

### Example 12 (powder properties)

The evaluation of powder properties, including angle of repose, bulk density and tap density, was carried out on the crystalline form I of example 2a and the amorphous sample of example 1a to understand the flow properties of crystalline form I and amorphous powder.

Bulk density and tap density: add a certain quality of the sample to be evaluated into a 5-ml graduated cylinder, and record the volume at this time. The bulk density is calculated by dividing the mass of the sample by the volume at this time; Tap the graduated cylinder 200 times and record the final volume. The tap density is obtained by dividing the sample mass by the final volume. Each parameter was tested three times in parallel.

Angle of repose: fix the funnel vertically with the bottom and slowly add the material into the funnel. The base forms a vertebral body of symmetrical material. The height and bottom diameter of the vertebral body were measured. Three times in parallel.

The results of bulk density / tap density are summarized in Table 17. The results showed that the average bulk density and tap density of crystalline form I were 0.34 g / cm3 and 0.46 g / cm3, respectively, and the calculated Karl index was 26%; The average bulk density and tap density of the amorphous are 0.31 g / cm3 and 0.46 g / cm3, respectively, and the calculated Karl index is 33%.

The results of angle of repose are summarized in table 18. The results showed that the repose angles of the crystalline form I and amorphous samples were 27.7 ° and 26.7 °, respectively. According to the comprehensive evaluation results, the crystalline form I and amorphous samples have relatively close Karl index and angle of repose, indicating that crystalline form I and amorphous have similar fluidity.

**Table 17 Bulk density and tap density test results**

| Number of samples | Test number | Bulk density (g/cm3) | Mean bulk density (g/cm3) | Tap density (g/cm3) | Mean tap density (g/cm3) | Karl index |
|---|---|---|---|---|---|---|
| Example 2a crystalline form I | 1 | 0.34 | 0.34 | 0.48 | 0.46 | 26% |
| | 2 | 0.35 | | 0.44 | | |
| | 3 | 0.34 | | 0.47 | | |
| Example 1a amorphous form | 1 | 0.30 | 0.31 | 0.46 | 0.46 | 33% |
| | 2 | 0.32 | | 0.48 | | |
| | 3 | 0.32 | | 0.43 | | |

| | | | | | | |
|---|---|---|---|---|---|---|
| Karl index= (tap density-bulk density)/tap density | | | | | | |

**Talbe 18 Angle of repose test results**

| Number of samples | Test number | vertebral body height(cm) | Vertebral body diameter (cm) | Angle of repose (°) | Mean angle of repose (°) |
|---|---|---|---|---|---|
| Crystalline Form I | 1 | 1.1 | 4.4 | 26.6 | 27.7 |
| | 2 | 1.1 | 4.3 | 27.1 | |
| | 3 | 1.3 | 4.6 | 29.5 | |
| amorphous form | 1 | 1.1 | 4.1 | 28.2 | 26.7 |
| | 2 | 0.9 | 3.6 | 26.6 | |
| | 3 | 0.9 | 3.8 | 25.4 | |

| | | | | | |
|---|---|---|---|---|---|
| Angle of repose α Calculation formula: α = tan⁻¹ (h/D), h: vertebral body height, D: Vertebral body diameter | | | | | |

### Example 13(Mechanical force stability)

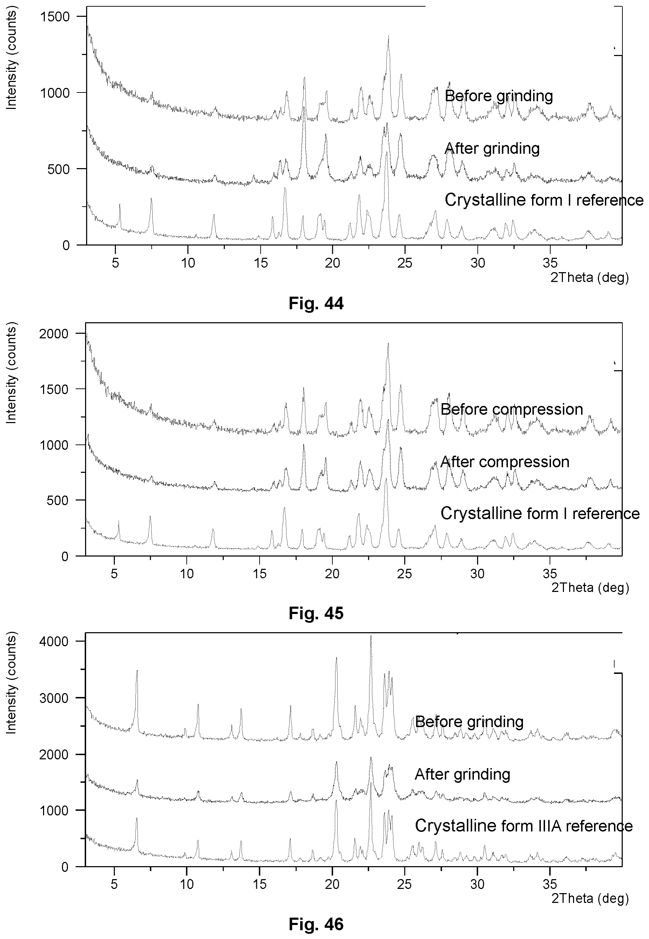

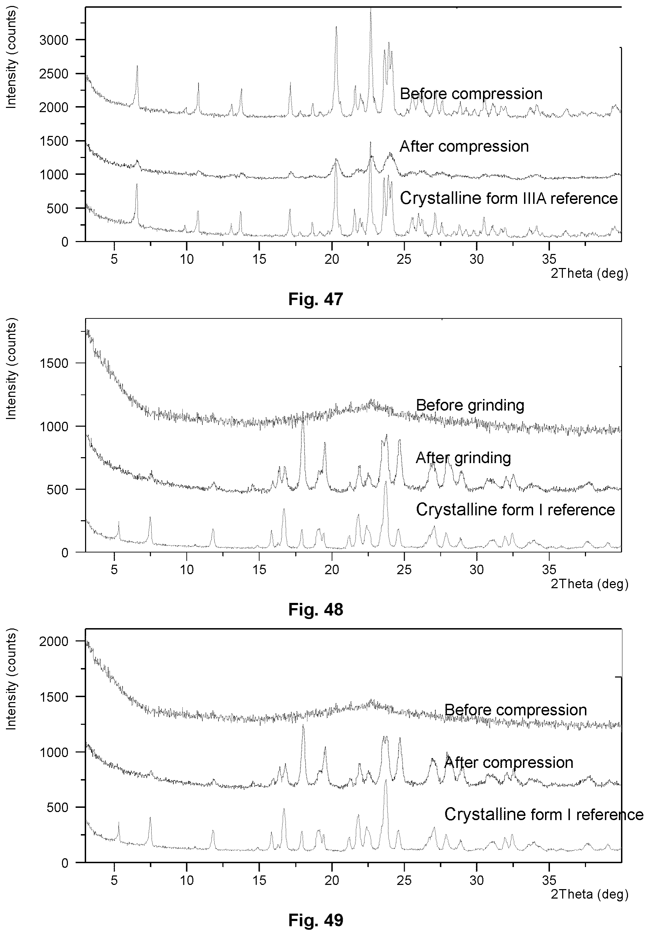

The crystal I of Example 2a, the crystal IIIA of Example 4a and the amorphous form of Example 1a were manually ground and tableted (350 MPa pressure), and the ground and tableted samples were subjected to XRPD testing to evaluate their mechanical stability. XRPD results are shown in Fig. 44, Fig. 45, Fig. 46, Fig. 47, Fig. 48, and Fig. 49. The evaluation results showed that there was no crystal transformation and no significant crystallinity reduction of crystalline form I after grinding and tablet pressing. After grinding, the crystalline form III remained unchanged but the crystallinity decreased slightly. After pressing, the crystalline form III remained unchanged but the crystallinity decreased significantly. The amorphous form is converted to form I after grinding and tabbing.

According to the crystalline form
characterization and identification results, the crystalline form I was selected for the evaluation of moisture conduction, solid stability, solubility, powder properties and mechanical stability. At the same time, the amorphous form was also evaluated to compare its properties with the crystalline form I. The results of DVS showed that the crystalline form I had almost no moisture diversion, and no crystal transformation occurred after DVS testing. The amorphous form was converted to form I after DVS testing. Solid state stability results showed that no crystal transformation or purity reduction occurred after closed storage at 60 °C for 1 day and open storage at 25 °C/60%RH and 40 °C/75%RH for 1 week, indicating that the crystalline form I had good physical and chemical stability under the evaluated conditions. The amorphous forms showed no significant change in purity under the three evaluated conditions, but all changed to form I. The results of the chemical properties test of the crystal I powder show that the crystal I and the amorphous sample have similar fluidity. The mechanical stability results showed that after tabulation (350 MPa) and manual grinding (about 3 min), the amorphous form changed to crystalline form I without significant crystal transformation and crystallinity reduction.

Based on the characterization data and evaluation results, the crystalline form I did not undergo a crystal transition under all evaluated conditions, while the amorphous form changed to crystal I after DVS, solid-state stability, solubility, and mechanical force stability tests, indicating that the crystalline form I has better physical stability than the amorphous form.

### Example 14(Light stability)

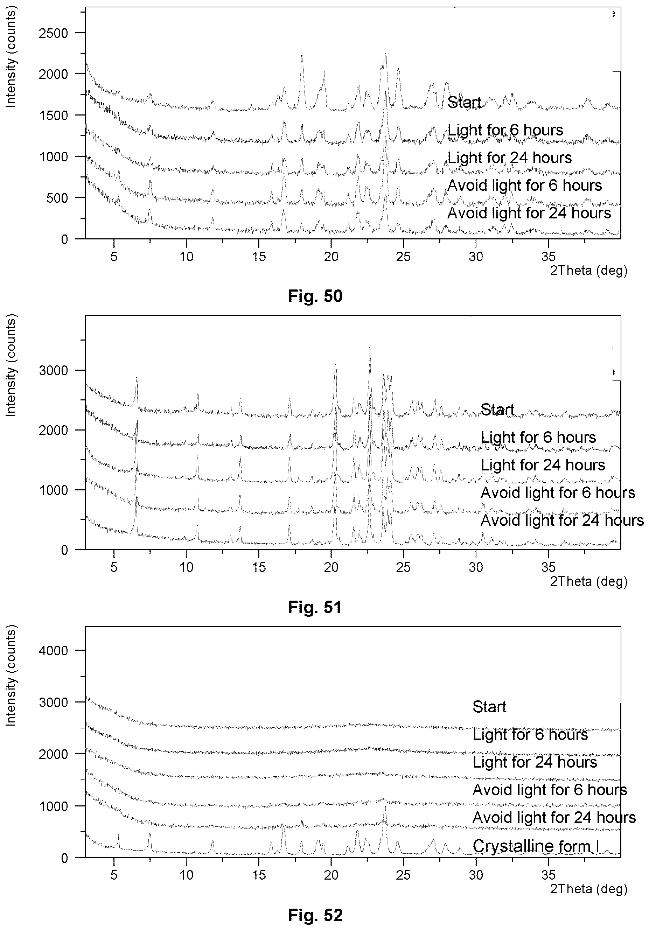

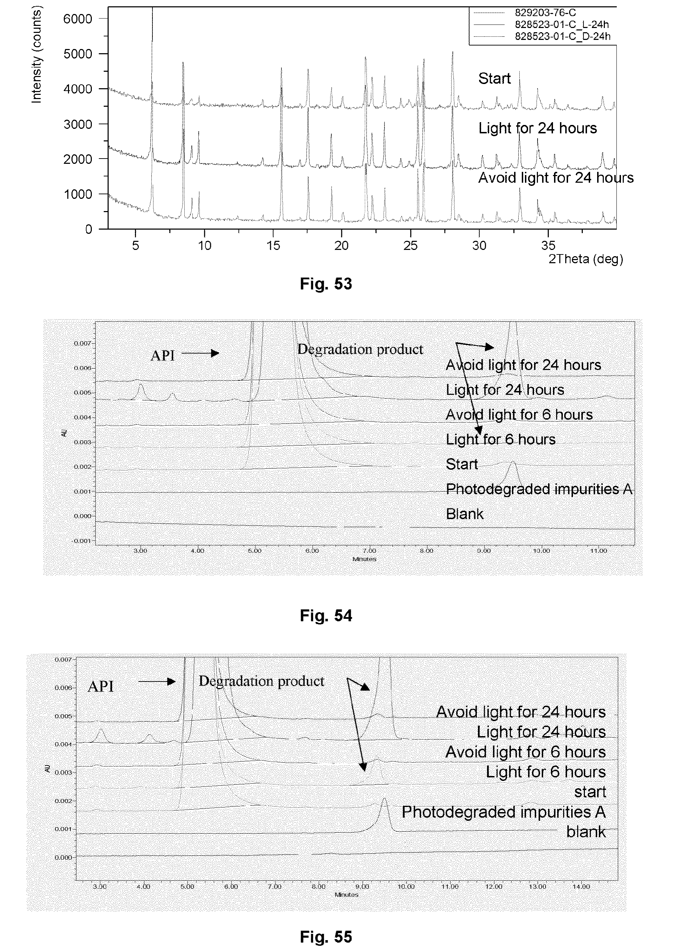

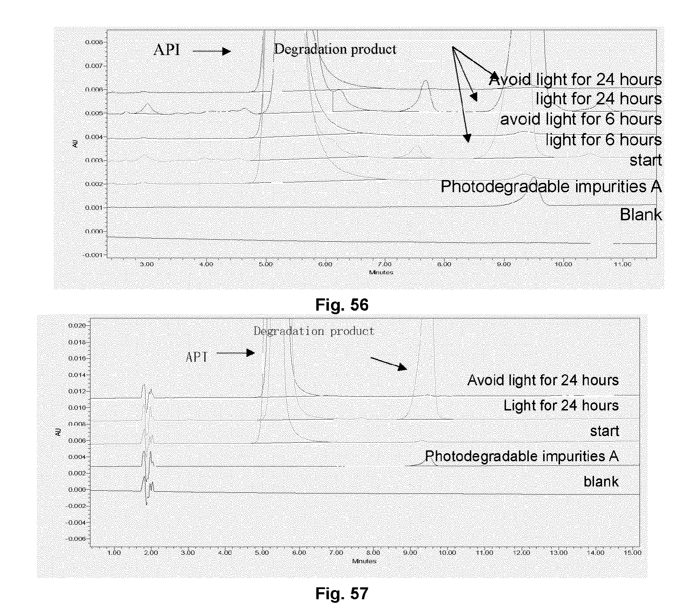

"For crystalline form I of Example 2a, crystalline form IIIA of Example 4a, crystalline form V of example 6a and amorphous form of Example 1a, some samples were taken at 6 hours and 24 hours under light (white light 5800 to 5890 Lux + UV 7.9 to 8.7 W/m2) for XRPD and HPLC purity testing." Stability assessment was performed. The XRPD results are shown in Fig. 50, Fig. 51, Fig. 52, and Fig. 53: no crystal transformation occurred in the crystalline forms I, IIIA, V, and amorphous forms after being placed under light conditions for 24 hours. Under reference conditions in the dark, no transcrystallization occurred after 24 hours of storage for crystal I, crystal IIIA, and crystal V, and a faint diffraction peak of crystal I was observed after 6 and 24 hours of amorphous storage. The HPLC results are shown in Fig. 54, Fig. 55, Fig. 56, Fig. 57 and Table 19: After 24 hours of light exposure, the purity of crystal I decreased slightly from 100.00 area % to 99.14 area % (the content of impurity A was 0.79%) under light exposure. The purity of crystalline form IIIA, crystalline form V and amorphous form decreased from 100.00 area % to 98.46 area % (impurity A content 1.34%), 92.05 area % (impurity A content 7.95%) and 90.42 area % (impurity A content 8.83%) under light condition, respectively. There was no significant change in the purity of the four samples under the reference conditions in the dark (the samples exposed to light for 6 hours in Fig. 54, Fig. 55 and Fig. 56 were tested separately from the photodegraded impurities in two HPLC test sequences, so the retention time of impurities may be slightly offset). According to the results of light stability, the crystalline form I exhibits better light stability compared to the crystalline form IIIA, V and amorphous form.

**Table 19 Results of light stability of the crystalline forms I/IIIA/V and the amorphous form**

| Crystalline Form (Sample No.) | Light condition | Point of sampling | Crystalline form change | Purity (area%) |
|---|---|---|---|---|
| Crystalline Form I | -- | 0 hours | -- | 100.00 |
| | light | 6 hours | no | 99.95 |
| | | 24 hours | no | 99.14 |
| | Avoid light | 6 hours | no | 100.00 |
| | | 24 hours | no | 100.00 |
| Crystalline Form IIIA | -- | 0 hours | -- | 100.00 |
| | light | 6 hours | no | 99.75 |
| | | 24 hours | No | 98.46 |
| | Avoid light | 6 hours | No | 100.00 |
| | | 24 hours | No | 100.00 |
| amorphous form | -- | 0 hours | -- | 100.00 |
| | light | 6 hours | No | 95.90 |
| | | 24 hours | No | 90.42 |
| | Avoid light | 6 hours | Yes | 100.00 |
| | | 24 hours | Yes | 99.94 |
| Crystalline Form V | -- | 0 hours | -- | 100.00 |
| | light | 24 hours | No | 92.05 |
| | Avoid light | 24 hours | no | 100.00 |

### Example 15 (Pharmacokinetic experiments in SD rats)

2.39 mg of crystalline form I of Example 2a, 2.38 mg of crystalline form IIIA of Example 4a, and 2.46 mg of amorphous form of Example 1 were used as test materials, and 7.967mL, 7.906 mL, and 8.193 mL of 0.5% CMC-Na aqueous solution were added, respectively. Formulations were prepared for intragastric administration at a final concentration of 0.3 mg/mL. Three male Sprague-Dawley (SD) rats were given the three test solutions by gavage at a dose of 3 mg/kg of animal body weight, respectively. Blood samples of 0.15mL were collected from the jugular sinus at 0, 0.25h, 0.5h, 1h, 2h, 4h, 8h, 10h, and 24h. The collected whole blood was placed in EDTA-K2 anticoagulant tubes, mixed thoroughly, centrifuged (1500~1600 g) for 10 min, and the plasma was separated and used for biological analysis. LC-MS/MS analysis method (instrument model: Triple Quad 5500; Agilent ZORBAX XDB-C18 column; Flow rate: 0.50 mL/min; Injection volume: 2 µL; Mobile phase A: water [0.1% formic acid + 5mM ammonium acetate], mobile phase B: acetonitrile [0.1% formic acid]) were used to determine the concentration of the test substance in plasma samples. The corresponding pharmacokinetic parameters were calculated with the use of the noncompartmental model in Pharsight Phoenix 8.3 (Table 20). According to the data, the AUC₀₋ₜ of form I and form IIIA were 9820 ng·h/mL and 8890 ng·h/mL, respectively, and Cₘₐₓ were 1160ng/mL and 1030 ng/mL, respectively, indicating adequate drug plasma exposure in vivo and better pharmacokinetic properties. Crystalline form I possesses a higher exposure and blood concentration than crystalline form IIIA, which is shown to have the potential to reduce the dose.

**Table 20 Pharmacokinetic parameters of crystalline form I, IIIA and amorphous form**

| | | Crystalline form I | Crystalline form III A | amorphous form |
|---|---|---|---|---|
| T_{1/2} | h | 3.37 | 2.99 | 2.99 |
| Tₘₐₓ | h | 4.00 | 2.00 | 4.00 |
| Cₘₐₓ | ng/mL | 1160 | 1030 | 1800 |
| AUC₀₋ₜ | ng·h/mL | 9730 | 8840 | 13800 |
| AUC_{0-inf} | ng·h/mL | 9820 | 8890 | 13900 |
| MRT₀₋ₜ | h | 5.93 | 5.42 | 5.67 |
| MRT_{0-inf} | h | 6.16 | 5.54 | 5.80 |

### Example 16: Inhibition of proliferation of a melanoma cell line with NRAS mutation

All cells were provided by Beijing Cancer Hospital. Human melanoma cell line SK-MEL-2 (NRAS Q61R) was cultured with MEM supplemented with 10%FBS and penicillin streptomycin in an incubator at 37 °C and 5% carbon dioxide. Human melanoma cell line HMVII (NRAS Q61K) was cultured with F12K supplemented with 10%FBS and penicillin streptomycin at 37 °C in an incubator with 5% carbon dioxide.

When the confluence of the cultured cells reached more than 80%, the adherent cells were digested with trypsin and the cell precipitate was collected by centrifugation, counted, and 90 µL of the cell suspension was seeded in a 96-well plate at the appropriate density. After 24 hours, a series of gradient dilutions of the compound (crystalline form I) were added to each well in a volume of 10 µL (concentration range 0.15 nM-10 µM, 4-fold gradient dilution). Each concentration was set up three multiple Wells, a total of 9 concentration points. The Wells added with the same volume of 5% DMSO were used as control, and the final concentration of DMSO was 0.5%. After 3 days of drug treatment, the cell viability was detected by MTT, and 10 µL MTT was added to each well and incubated in the incubator for another 4 hours. After the supernatant was discarded and 150 µL DMSO was added to dissolve the crystal, and the absorbance at 490 nm was detected by microplate reader. The above experiments were repeated three times. The dose-effect curve was made and IC50 was calculated by GraphPad Prism 8 software. Results are presented as the mean IC50 ±SD of three experiments (Table 21 and 22).

**Table 21. IC₅₀ values of compound 1 on SK-MEL-2 cells**

| Compound | IC₅ₒ(nM) | | | Mean value (nM) | SD |
|---|---|---|---|---|---|
| | 1^{st} | 2^{nd} | 3^{rd} | | |
| Compound1 | 2.76 | 4.26 | 4.01 | 3.68 | 0.66 |
| MEK162 | 74.79 | 68.27 | 221.99 | 121.68 | 70.98 |

**Table 22. IC50 values of compound 1 on HMVII cells**

| Compound | IC₅₀(nM) | | | Mean value (nM) | SD |
|---|---|---|---|---|---|
| | 1^{st} | 2^{nd} | | | |
| compound 1 | 4.81 | 7.45 | 3.19 | 5.15 | 2.15 |

### Example 17: Inhibition of RAS or RAF mutations

MTS assay was used to detect the in vitro anti-proliferative activity of compound 1 on RAS mutant or RAF mutant tumor cell lines, RAS/RAF wild-type tumor cell lines, and normal human cell lines.

Adherent cells in logarithmic growth phase were digested with trypsin or suspended cells were collected by centrifugation, counted, and seeded in 150 µL in a 96-well plate. After 24 hours, 50 µL/ well of a 4-fold final concentration of compound (crystalline form I) diluted in medium was added (concentration range 0.15nM-1000nM, 3-fold gradient dilution). Wells in which the same volume of 2% DMSO was added to a final DMSO concentration of 0.5% were used as controls. After the cells continued to be culture for 72 hours, MTS was used to measure cell viability. The method was as follows: adherent cells medium was discarded, 20 µL MTS and 100 µL cell medium were added to each well, and 20 µL MTS was added directly to the suspended cells. The OD490 was detected after 1-4 hours of continuous culture in the incubator, and the OD650 value was used as a reference. The dose-effect curve was made by GraphPad Prism software and IC₅₀ was calculated. The results are shown in Table 23.

**Table 23. IC₅₀ values of compound 1 on RAS or RAF mutated tumor cell lines and human embryo lung cell line MRC-5**

| # | Cell lines | Ras/Raf status | IC₅₀ (nM) |
|---|---|---|---|
| 1 | A375 | Raf mutant | 0.86±0.07 |
| 2 | COLO-205 | Raf mutant | 0.94±0.26 |
| 3 | LOX | Raf mutant | 1067.99±324.79 |
| 4 | COLO-829 | Raf mutant | 3.46±0.27 |
| 5 | HT-29 | Raf mutant | 2.35±0.03 |
| 6 | Calu-6 | Ras mutant | 10.07±1.18 |
| 7 | A549 | Ras mutant | 59.89±11.06 |
| 8 | PANC-1 | Ras mutant | >1000 |
| 9 | HL-60 | Ras mutant | 0.67±0.28 |
| 10 | H1975 | Ras/Raf wt | >1000 |
| 11 | BxPC-3 | Ras/Raf wt | 252.04±81.39 |
| 12 | MRC-5 | Normal cells | >1000 |

### Example 18:

A multicenter, single-arm, phase II study to evaluate the efficacy and safety of compound 1, crystalline form I, in patients with advanced melanoma with NRAS mutations. Patients received 12mg twice daily until the occurrence of intolerable toxicity, disease progression (as assessed by the investigator according to RECIST 1.1), withdrawal, death, or a judgment by the investigator that the risks outmatched the benefits. By February 19, 2023, a total of 100 subjects were enrolled, of which 95.0% (95/100 cases) were included in the full analysis set (FAS).

### Primary efficacy measures:

In the FAS group, the ORR (objective response rate) assessed by IRRC was 35.8% (34/95 cases) (95%CI: 26.2%, 46.3%).

In the FAS group, the median PFS was 4.2 months (95%CI: 3.5, 5.6), the DCR was 72.6% (69/95 cases) (95%CI: 62.5%, 81.3%), and the median DoR was 6.1 months (95%CI: 3.9, 8.9).

Compound 1 had a good anti-tumor effect on advanced melanoma patients with NRAS mutation, and the ORR was 35.8% according to IRRC evaluation, which was significantly better than the clinical data of similar drugs.

## Claims

1. A polymorph of formula (I) 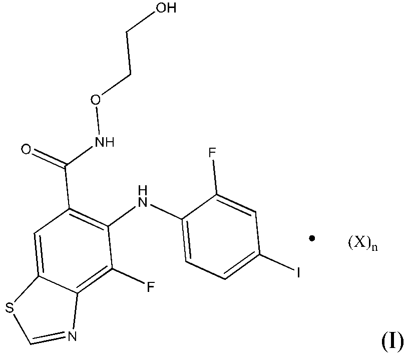 wherein n is 0 or 1, X is acetonitrile, water, 1,4-dioxane, ethanol, methanol, dimethylformamide, acetone or a mixture thereof.

2. The polymorph according to claim 1, wherein n is 0.

3. The polymorph according to claim 1, wherein n is 0; X is acetonitrile, water, 1,4-dioxane or ethanol.

4. The polymorph according to claim 1, wherein n is 0, and the polymorph is crystalline form I, **characterized in that** the X-ray powder diffraction pattern of the form I comprises characteristic diffraction peaks at the following 2θ positions: 16.71°±0.2°, 21.82°±0.2°, and 23.75°±0.2°, using Cu-Kα radiation.

5. The polymorph according to claim 4, wherein the X-ray powder diffraction pattern of the crystalline form I further comprises characteristic diffraction peaks at the following 2θ positions: 7.48°±0.2° and 22.36°±0.2°.

6. The polymorph according to claim 5, wherein the X-ray powder diffraction pattern of the crystalline form I further comprises characteristic diffraction peaks at the following 2θ positions: 5.3° ± 0.2°, 24.57°±02° and 27.08°±0.2°.

7. The polymorph according to claim 6, wherein the X-ray powder diffraction pattern of the crystalline form I further comprises characteristic diffraction peaks at the following 2θ positions: 11.81°±0.2°, 15.83°±0.2°, 17.92°±0.2°, 18.95°±0.2° and 19.17°±0.2°.

8. The polymorph according to claim 4, wherein the X-ray powder diffraction pattern of the crystalline form I comprises characteristic diffraction peaks at the following 2θ positions: 5.30 ° ± 0.2 °, 7.48 ° ± 0.2 °, 11.81 ° ± 0.2 °, 14.85 ° ± 0.2 °, 15.83 ° ± 0.2 °, 16.71 ° ± 0.2 °, 17.92 ° ± 0.2 °, 18.95 ° ± 0.2 °, 19.17 ° ± 0.2 °, 19.43 ° ± 0.2 °, 21.14 ° ± 0.2 °, 21.82 ° ± 0.2 °, 22.36 ° ± 0.2 °, 23.75 ° ± 0.2 °, 24.57 ° ± 0.2 °, 27.08 ° ± 0.2 °, 27.83 ° ± 0.2 °, 28.88 ° ± 0.2 °, 31.20 ° ± 0.2 °, 31.92 ° ± 0.2 °, 32.40 ° ± 0.2 °, 33.91 ° ± 0.2 °, 35.83 ° ± 0.2 ° 37.51 ° ± 0.2 ° and 39.04 ° ± 0.2 °.

9. The polymorph according to claim 4, wherein the X-ray powder diffraction pattern of the crystalline form I substantially as shown in Figure 3.

10. The polymorph according to claim 4, wherein the crystalline form I has a TGA thermogram and / or DSC thermogram as shown in Fig. 4.

11. The polymorph according to claim 1, wherein n is 1, X is acetonitrile, and the polymorph is crystalline form II, which is **characterized in that** the X-ray powder diffraction pattern of the crystalline form II comprises characteristic diffraction peaks at the following 2θ positions: 6.35 °±0.2 °, 20.34 °±0.2 °, 22.41 °±0.2 °, 22.6 °±0.2 °, 24.99 °±0.2 °, 26.05 °±0.2 °and 28.71 °±0.2 °, using Cu-Kα radiation.

12. The polymorph according to claim 11, wherein the X-ray powder diffraction pattern of the crystalline form II further comprises characteristic diffraction peaks at the following 2θ positions: 9.18 °±0.2 °, 16.03 °±0.2 °, 18.25 °±0.2 °, 27.07 °±0.2 °, 29.08 °±0.2 °and 33.93 °±0.2 °.

13. The polymorph according to claim 12, wherein the X-ray powder diffraction pattern of the crystalline form II further comprises characteristic diffraction peaks at the following 2θ positions: 14.44 °±0.2 °, 24.64 °±0.2 °, 26.41 °±0.2 °, 32.27 °±0.2 °, 32.68 °±0.2 °, 37.07 °±0.2 °and 39.51 °±0.2 °.

14. The polymorph according to claim 11, The X-ray powder diffraction pattern of the crystalline form II comprises characteristic diffraction peaks at the following 2θ positions: 6.35 °±0.2 °, 9.18 °±0.2 °, 9.91 °±0.2 °, 14.44 °±0.2 °, 16.03 °±0.2 °, 18.25 °±0.2 °, 19.77 °±0.2 °, 20.34 °±0.2 °, 21.81 °±0.2 °, 22.41 °±0.2 °, 22.60 °±0.2 °, 23.84 °±0.2 °, 24.64 °±0.2 °, 24.99 °±0.2 °, 25.43 °±0.2 °, 26.05 °±0.2 °, 25.43 °±0.2 °, 26.05 °±0.2 °, 26.41 °±0.2 °, 27.07 °±0.2 °, 28.71 °±0.2 °, 29.08 °±0.2 °, 29.81 °±0.2 °, 31.16 °±0.2 °, 31.57 °±0.2 °, 32.27 °±0.2 °, 32.68 °±0.2 °, 33.93 °±0.2 °, 34.19 °±0.2 °, 35.42 °±0.2 °, 37.07 °±0.2 °, 37.56 °±0.2 °, 38.69 °±0.2 °and 39.51 °±0.2 °.

15. The polymorph according to claim 11, wherein the X-ray powder diffraction pattern of the crystalline form II is substantially as shown in Fig. 6.

16. The polymorph according to claim 11, wherein the crystalline form II has a TGA thermogram and / or DSC thermogram as shown in Fig. 7.

17. The polymorph according to claim 1, Wherein n is 0, and the polymorph is crystalline form IIIA, which is **characterized in that** the X-ray powder diffraction pattern of the form IIIA comprises characteristic diffraction peaks at the following 2θ positions: 6.59 °±0.2 °, 22.69 °±0.2 °, 20.32 °±0.2 °, 23.62 °±0.2 °, 23.91 °±0.2 °and 24.15 °±0.2 °, using Cu-Kα radiation.

18. The polymorph according to claim 17, wherein the X-ray powder diffraction pattern of the crystalline form IIIA further comprises characteristic diffraction peaks at the following 2θ positions: 10.8 °±0.2 °, 17.14 °±0.2 °, 13.75 °±0.2 °, 21.59 °±0.2 °and 26.01 °±0.2 °.

19. The polymorph according to claim 18, wherein the X-ray powder diffraction pattern of the crystalline form IIIA further comprises the characteristic diffraction peaks at the following 2θ positions: 18.71 °±0.2 °, 21.97 °±0.2 °, 25.54 °±0.2 °, 27.13 °±0.2 °, 27.59 °±0.2 °and 30.51 °±0.2 °.

20. The polymorph according to claim 17, wherein the X-ray powder diffraction pattern of the crystalline form IIIA comprises the characteristic diffraction peaks at the following 2θ positions: 6.59 °±0.2°, 9.9° ±0.2°, 10.8° ±0.2°, 13.09 °±0.2°, 13.75° ±0.2°, 17.14° ±0.2°, 17.87 °±0.2°, 18.71° ±0.2°, 19.19 °±0.2°, 20.32 °±0.2°, 21.59 °±0.2°, 21.97 °±0.2°, 22.69 °±0.2°, 23.62° ±0.2°, 23.91° ±0.2°, 24.15 °±0.2°, 25.54 °±0.2 °, 26.01 °±0.2 °, 27.13 °±0.2 °, 27.59 °±0.2 °, 28.83 °±0.2 °, 29.24 °±0.2 °, 30.51 °±0.2 °, 31.13 °±0.2 °, 31.79 °±0.2 °, 33.6 °±0.2 °, 34.14 ±0.2 °, 36.08 °±0.2 °, 36.67 °±0.2 °and 37.26 °±0.2 °.

21. The polymorph according to claim 17, wherein the X-ray powder diffraction pattern of the crystalline form IIIA is substantially as shown in Fig. 9.

22. The polymorph according to claim 17, wherein the crystalline form IIIA has a TGA thermogram and / or DSC thermogram as shown in Fig. 10.

23. The polymorph according to claim 1, wherein n is 0, the polymorph is crystalline form IIIB, which is **characterized in that** the X-ray powder diffraction pattern of the crystalline form IIIB comprises characteristic diffraction peaks at the following 2θ positions: 6.53 °±0.2 °, 13.69 °±0.2 °, 18.6 °±0.2 °, 20.19 °±0.2 °, 21.52 °±0.2 °and 22.64 °±0.2 °, using Cu-Kα radiation.

24. The polymorph according to claim 23, wherein the X-ray powder diffraction pattern of the crystalline form IIIB further comprises characteristic diffraction peaks at the following 2θ positions: 10.75 °±0.2 °, 17.07 °±0.2 °, 21.93 °±0.2 °, 26.13 °±0.2 °, 23.57 °±0.2 °and 30.46 °±0.2 °.

25. The polymorph according to claim 24, wherein the X-ray powder diffraction pattern of the crystalline form IIIB further comprises characteristic diffraction peaks at the following 2θ positions: 13.05 ±0.2 °, 16.63 °±0.2 °, 20.82 °±0.2 °, 24.01 °±0.2 °, 27.55 °±0.2 °, 31.79 °±0.2 °.

26. The polymorph according to claim 23, wherein the X-ray powder diffraction pattern of the crystalline form IIIB comprises characteristic diffraction peaks at the following 2θ positions: 6.53 ° ± 0.2 °, 10.75 ° ± 0.2 °, 12.62 ° ± 0.2 °, 13.05 ° ± 0.2 °, 13.69 ° ± 0.2 °, 16.63 ° ± 0.2 °, 17.07 ° ± 0.2 °, 18.60 ° ± 0.2 °, 19.59 ° ± 0.2 °, 20.19 ° ± 0.2 °, 20.82 ° ± 0.2 °, 21.52 ° ± 0.2 °, 21.93 ° ± 0.2 °, 22.64 ° ± 0.2 °, 23.57 ° ± 0.2 °, 24.01 ° ± 0.2 °, 25.46 ° ± 0.2 °, 26.13 ° ± 0.2 °, 27.55 ° ± 0.2 °, 30.46 ° ± 0.2 °, 31.04 ° ± 0.2 °, 31.79 ° ± 0.2 °, 32.81 ° ± 0.2 ° 33.54 ° ± 0.2 °, 34.06 ° ± 0.2 °, and 34.46 ° ± 0.2 °.

27. The polymorph according to claim 23, wherein the X-ray powder diffraction pattern of the crystalline form IIIB is substantially as shown in Fig. 13.

28. The polymorph according to claim 23, wherein the crystalline form II has a TGA thermogram and / or DSC thermogram as shown in Fig. 14.

29. The polymorph according to claim 1, wherein n is 1 and X is 1,4-dioxane, and the polymorph is crystalline form IV, which is **characterized in that** the X-ray powder diffraction pattern of the crystalline form IV comprises characteristic diffraction peaks at the following 2θ positions: 8.56 °±0.2 °, 13.29 °±0.2 °, 17.69 °±0.2 °, 19.75 °±0.2 °and 22.45 °±0.2 °, using Cu-Kα radiation.

30. The polymorph according to claim 29, wherein the X-ray powder diffraction pattern of the crystalline form IV further comprises characteristic diffraction peaks at the following 2θ positions: 5.26 °±0.2 °, 18.29 °±0.2 °, 31.83 °±0.2 °, 25.68 °±0.2 °, 22.86 °±0.2 °, 32.81 °±0.2 °and 23.44 °±0.2 °.

31. The polymorph according to claim 30, wherein the X-ray powder diffraction pattern of the crystalline IV further comprises characteristic diffraction peaks at the following 2θ positions: 126.57 °±0.2 °, 27.52 °±0.2 °, 35.69 °±0.2 °, 21.09 °±0.2 °, 20.35 °±0.2 °and 31.43 °±0.2 °.

32. The polymorph according to claim 29, wherein the X-ray powder diffraction pattern of the crystalline form IV comprises characteristic diffraction peaks at the following 2θ positions: 5.26 °±0.2 °, 8.56 °±0.2 °, 9.85 °±0.2 °, 13.29 °±0.2 °, 17.69 °±0.2 °, 18.29 °±0.2 °, 19.75 °±0.2 °, 20.35 °±0.2 °, 21.09 °±0.2 °, 22.45 °±0.2 °, 22.86 °±0.2 °, 23.44 °±0.2 °, 24.44 °±0.2 °, 25.68 °±0.2 °, 26.57 °±0.2 °, 27.52 °±0.2 °, 24.44 °±0.2 °, 25.68 °±0.2 °, 26.57 °±0.2 °, 27.52 °±0.2 °, 28.40 °±0.2 °, 29.78 °±0.2 °, 31.43 °±0.2 °, 31.83 °±0.2 °, 32.81 °±0.2 °, 34.29 °±0.2 °, 35.69 °±0.2 °and 37.72 °±0.2 °.

33. The polymorph according to claim 29, wherein the X-ray powder diffraction pattern of the crystalline form IV is substantially as shown in Fig. 17.

34. The polymorph according to claim 29, wherein the crystalline form IV has a TGA thermogram and / or DSC thermogram as shown in Fig. 18.

35. The polymorph according to claim 1, wherein n is 1 and X is 1,4-dioxane, and the polymorph is crystalline form V, which is **characterized in that** the X-ray powder diffraction pattern of the crystalline form V comprises characteristic diffraction peaks at the following 2θ positions: 6.21 °±0.2 °, 8.47 °±0.2 °, 15.62 °±0.2 °, 21.73 °±0.2 °, 25.53 °±0.2 °, 25.94 °±0.2 °and 28.05 °±0.2 °, using Cu-Kα radiation.

36. The polymorph according to claim 35, wherein the X-ray powder diffraction pattern of the crystalline form V further comprises characteristic diffraction peaks at the following 2 θ positions: 9.61 °±0.2 °, 17.55 °±0.2 °, 19.25 °±0.2 °, 22.22 °±0.2 °, 23.12 °±0.2 °, 32.92 °±0.2 °and 34.22 °±0.2 °.

37. The polymorph according to claim 36, wherein the X-ray powder diffraction pattern of the crystalline form V further comprises characteristic diffraction peaks at the following 2θ positions: 9.06 °±0.2 °, 20.07 °±0.2 °, 28.49 °±0.2 °, 30.21 °±0.2 °, 31.25 °±0.2 °, 35.47 °±0.2 °and 38.94 °±0.2 °.

38. The polymorph according to claim 35, wherein the X-ray powder diffraction pattern of the crystalline form V further comprises characteristic diffraction peaks at the following 2θ positions: 6.21 °±0.2 °, 8.47 °±0.2 °, 25.94 °±0.2 °, 15.62 °±0.2 °, 25.53 °±0.2 °, 28.05 °±0.2 °, 21.73 °±0.2 °, 17.55 °±0.2 °, 32.92 °±0.2 °, 23.12 °±0.2 °, 22.22 °±0.2 °, 19.25 °±0.2 °, 34.22 °±0.2 °, 9.61 °±0.2 °, 9.06 °±0.2 °, 38.94 °±0.2 °, 34.22 °±0.2 °, 9.61 °±0.2 °, 9.06 °±0.2 °, 38.94 °±0.2 °, 31.25 °±0.2 °, 35.47 °±0.2 °, 28.49 °±0.2 °, 30.21 °±0.2 °, 20.07 °±0.2 °, 39.78 °±0.2 °, 14.26 °±0.2 °, 24.32 °±0.2 °, 16.95 °±0.2 °, 32.33 °±0.2 °, 36.43 °±0.2 °, 24.94 °±0.2 °, 12.42 °±0.2 °and 37.86 °±0.2 °.

39. The polymorph according to claim 35, wherein the X-ray powder diffraction pattern of the crystalline form V is substantially as shown in Fig. 20.

40. The polymorph according to claim 35, wherein the crystalline form V has a TGA thermogram and / or DSC thermogram as shown in Fig. 21.

41. The polymorph according to any one of claims 1-40, wherein the polymorph is substantially free of impurities (A).

42. The polymorph according to claim 40, wherein the polymorph of compound 1 contains less than 0.15% by weight of impurities (A) relative to the polymorph.

43. The polymorph according to claim 4, wherein the crystalline form I is substantially free of impurities (A).

44. The polymorph according to claim 43, wherein the crystalline form I contains less than 0.15% by weight of impurities (A) relative to the crystalline form I.

45. A pharmaceutical composition comprising a polymorphic form of any one of claims 1-44, as well as a pharmaceutically acceptable carrier and/or excipient.

46. The pharmaceutical composition according to claim 45, wherein the polymorph is crystalline form I.

47. A method for treating mammalian tumors, chronic inflammatory diseases, inflammatory intestinal diseases, skin diseases, diabetes, eye diseases, diseases associated with mammalian angiogenesis or angiogenesis, diseases associated with chronic pain, and other diseases modulated by Mek cascade, the method comprises administering the polymorph of any one of claims 1-44 to the mammalian.

48. The polymorph according to any of claims 1-44, which is used for the treatment of tumors in mammals, chronic inflammatory diseases, inflammatory intestinal diseases, skin diseases, diabetes, eye diseases, diseases associated with angiogenesis or angiogenesis in mammals, diseases associated with chronic pain, and other diseases modulated by Mek cascades.

49. The use of the polymorph of any one of claims 1-44 in preparing for the treatment of tumors, chronic inflammatory diseases, inflammatory intestinal diseases, skin diseases, diabetes, eye diseases, diseases associated with angiogenesis or angiogenesis of mammals, diseases associated with chronic pain, and other diseases modulated by Mek cascade.

50. A method for preparing crystalline form I of claim 4, comprising any one of the following:
a) the amorphous sample of compound 1 is added to the solvent, and then heated at a temperature above about 70 ° C, the resulting clear liquid is cooled to room temperature, which is kept at room temperature to continue stirring, solid precipitation, filtration, drying; or
b) The amorphous sample of compound 1 is dissolved in a good solvent, filtered to obtain a clear solution, stir the clear solution while adding an anti-solvent until solid precipitated; or
c) the amorphous sample of compound 1 is dissolved in a solvent, stirred at about 50 °C, then filter out the filtrate, cool the resulting filtrate to about 5 °C, and collect the precipitated solid.

51. The method according to claim 50, wherein the heating temperature in method 1) ranges from approximately 75 ° C to approximately 100 ° C; About 80 ° C to about 90 ° C; Or about 75 ° C or about 85 ° C.

52. The method according to claim 50, wherein the room temperature in method 1) is approximately 20-25 ° C.

53. The method according to claim 50, wherein the time for further stirring in method 1) is approximately 1-12 hours, approximately 1-8 hours, approximately 1-5 hours or longer, or approximately 24-96 hours or longer.

54. The method according to claim 50, wherein the clear liquid in method 1) is cooled to room temperature within about 2 to 5 hours or about 2.5 to 3 hours.

55. The method according to claim 50, wherein method 1) after the room temperature constant temperature continues stirring, the temperature can be optionally further reduced to about 0-10 ° C, constant temperature stirring.

56. The method according to claim 50, wherein stirring at about 0-10 ° C for about 1-12 hours, about 1-8 hours, about 1-5 hours or more.

57. The method according to claim 50, wherein the solvent in method 1) is water, methanol, ethanol, isopropanol, acetone, methyl isobutyl ketone, 2-butanone, ethyl acetate, isopropyl acetate, methyl tert-butyl ether, tetrahydrofuran, benzyl ether, 2-methyltetrahydrofuran, cyclopentyl methyl ether, 1,4-dioxane, acetonitrile, dichloromethane, toluene, m-xylene, n-hexane, n-pentane, dimethyl sulfoxide, dimethylacetamide N-methylpyrrolidone or its mixture.

58. The method according to claim 50, wherein the solvent in method 1) is ethanol.

59. The method according to claim 50, wherein the good solvent in method 2) is a solvent in which compound 1 is soluble, and the anti solvent in method 2) is a solvent in which compound 1 is insoluble.

60. The method of claim 50, wherein the good solvent in method 2) is MEK, 1-dioxane or DMSO; The antisolvent in method 2) is MTBE, EtOAc, CHCl₃, n-heptane, Anisole, EtOAc, _{H2O}, IPAc, CPME, DCM or toluene.

61. The method according to claim 50, wherein the cooling rate of method 3) is 0.1 ° C / min.

62. The method according to claim 50, wherein the solvent of method 3) is MIBK, Methyl acetate, 2-MeTHF, or acetone/EtOH (1:1).

63. A method for preparing the crystalline form V of claim 35, wherein the method comprises: The amorphous sample of compound 1 is added to the solvent and then heated at a temperature below about 70 ° C, and the obtained clear liquid is cooled to about 0- 10 ° C, stayed at that temperature until the solid is precipitated, dried.

64. The method according to claim 63, wherein the method is heated at about 70 ° C, about 60 ° C or about 50 ° C.

65. The method according to claim 63, wherein the cooling rate is approximately 0.1-0.5 ° C/min.

66. The method according to claim 63, wherein the cooling rate is approximately 0.1 ° C/min.

67. The method according to claim 63, wherein the obtained clear liquid is cooled to about 5 ° C.

68. A method for treating mammalian RAS or RAF mutant cancers, wherein the method comprises administering 4-fluoro-5- (2-fluoro-4-iodophenylamino) -1H- benzo[d] thiazole-6-carboxylic acid (2-hydroxy-ethoxy) - amide (compound 1) or its pharmaceutically acceptable salt to the mammalian.

69. The method according to claim 68, wherein the RAS or RAF mutant cancer is, such as, KRAS mutant cancer, NRAS mutant cancer, HRAS mutant cancer or BRAF mutant cancer.

70. The method according to claim 68 or 69, wherein the RAS mutant cancer is pancreatic cancer, colorectal cancer, lung cancer, melanoma, acute myeloid leukemia, bladder cancer or head and neck cancer, etc.

71. The method according to claims 68 to 70, wherein the cancer is a NRAS mutant cancer.

72. The method according to claim 71, wherein the NRAS mutant cancer is a NRAS mutated melanoma.

73. The method according to claim 72, wherein KRAS comprises mutations at one or more positions selected from codons 12, 13, 59, and 61.

74. The method according to claims 68 to 70, wherein NRAS comprises mutations at one or more positions selected from codons 12, 13, 59, 61, and 146.

75. The method according to claims 68 to 70, wherein the NRAS mutation form has mutations at one or more amino acid positions selected from G12, G13, A59, Q61, K117, and A146.

76. The method according to claim 75, wherein the NRAS mutant form has one or more amino acid substitutions selected from: G12C, G12R, G12S, G12A, G12D, G12V, G13C, G13R, G13S, G13A, G13D, G13V, A59D, A59T, Q61K, Q61L, Q61R, Q61H, K117N, K117R, K117E, A146P, A146T, and A146V.

77. The method according to claims 68 to 70, wherein the cancer is an early, middle or advanced stage cancer. Cancer can be locally advanced or metastatic.

78. The method according to claims 68 to 70, wherein the mammal has previously received immunotherapy.

79. The method according to claim 78, wherein the mammal has previously received immunotherapy and suffers from advanced melanoma with NRAS mutation.

80. The method according to claims 68 to 70, wherein the melanoma is selected from: advanced melanoma, unresectable melanoma, metastatic melanoma, melanoma with BRAF mutation, melanoma with NRAS mutation, skin melanoma, or intraocular melanoma.

81. The method according to claims 68 to 80, wherein compound 1 is in the form of a capsule.

82. The method according to claim 81, wherein compound 1 is administered in a dose of 5-50mg once or twice a day.

83. The method according to claim 82, wherein compound 1 is administered in a twice-daily dose of 12mg each time.

84. The method according to claims 68 to 83, wherein the compound 1 is any one of the polymorphic forms from crystalline form I to crystalline form VI.

85. The method according to claims 68 to 83, wherein the compound 1 is crystalline form I.
